# EUROPEAN PATENT SPECIFICATION

(11) **EP 3 036 216 B1**
(45) Date of publication and mention of the grant of the patent: **12.06.2019**
(21) Application number: 14757880.1
(22) Date of filing: 20.08.2014
(51) Int. Cl.: C07C 253/30, C07C 255/07

(54) **PROCESS FOR THE MANUFACTURE OF HYDROGENATED NITRILES**
VERFAHREN ZUR HERSTELLUNG VON HYDRIERTEN NITRILEN
PROCESSUS DE FABRICATION DE NITRILES HYDROGÉNÉS

(30) Priority: 20.08.2013 EP 13181064; 20.08.2013 US 201361867719 P; 20.08.2013 EP 13181059; 20.08.2013 US 201361867815 P; 20.08.2013 EP 13181061; 20.08.2013 US 201361876782 P; 18.09.2013 US 201361879382 P
(43) Date of publication of application: 29.06.2016
(73) Proprietor: DSM IP Assets B.V., 6411 TE Heerlen (NL)
(72) Inventor: BONRATH, Werner, CH-4002 Basel (CH); MEDLOCK, Jonathan, CH-4002 Basel (CH); LEHMANN, Hajo, CH-4002 Basel (CH); BEUMER, Raphael, CH-4002 Basel (CH); FISCHESSER, Jocelyn, CH-4002 Basel (CH)
(74) Representative: Steck, Melanie
(86) International application number: PCT/EP2014/067765
(87) International publication number: WO 2015/024978

(56) References cited:
- US-A1- 2008 177 100

## Description

The present invention is directed to a process for the manufacture of a compound of formula (II) 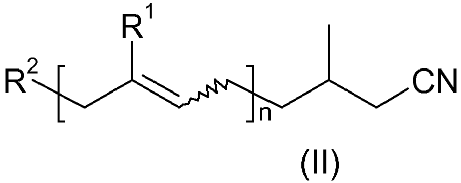 by reduction of a compound of formula I 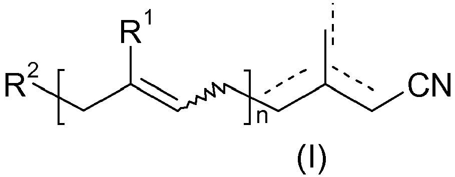 with hydrogen in the presence of a catalyst comprising nickel, wherein an additive is present, and
wherein n is either 1 or 2, preferably wherein n = 1;
wherein R¹ is linear C₁₋₄ alkyl or branched C₃₋₄ alkyl,
preferably wherein R¹ is methyl or ethyl, and
wherein R² is hydrogen or linear C₁₋₄ alkyl or branched C₃₋₄ alkyl,
preferably wherein R² is hydrogen or methyl, and
more preferably wherein either R¹ is methyl or R² is hydrogen;
wherein the additive is selected from the group of additives containing a nitrogen or a sulfur atom,
wherein the additive containing a sulfur atom is selected from the group of aromatic disulfides, aliphatic disulfides, aromatic sulfides, heteroaromatic sulfides, aliphatic sulfides, aromatic thiols, heteroaromatic thiols, aliphatic thiols, each containing a total number of 2-15 carbon atoms and a total number of 1 or 2 sulfur atoms, respectively, and wherein the additive containing a nitrogen atom is selected from the group of aliphatic primary amines containing a total number of 3-10 carbon atoms and 1-3 nitrogen atoms. and C6-C10 bicyclic aliphatic amidines; and wherein the reduction with hydrogen is carried out at an absolute pressure in the range of 1 to 20 bar.

The reduction with hydrogen (the hydrogenation) of the compound of formula (I) is carried out in an organic solvent or a mixture of organic solvents or without using any solvent.

One embodiment of the present invention is a solvent-free hydrogenation of the compound of formula (I). "Solvent-free" means without the usual amounts of such solvents. It is meant that no solvent is added to the starting materials, the compounds of formula (I).

But it is possible that the compounds of formula (I) may comprise traces of a solvent, which can originate from their production. But the amount of such solvent impurities is small, less than 10 weight-% and generally less than 2 weight-%.

### Solvent

If the hydrogenation is carried out in an organic solvent, the organic solvent is preferably selected from the group consisting of
◊ aliphatic hydrocarbons (preferred: linear, branched or cyclic aliphatic hydrocarbons having 5 - 10 carbon atoms; more preferred: linear aliphatic hydrocarbons having 5 - 10 carbon atoms, i.e. n-pentane, n-hexane, n-heptane, n-octane, n-nonane and n-decane; even more preferred: linear aliphatic hydrocarbons having 6 - 8 carbon atoms, i.e. n-hexane, n-heptane, n-octane; most preferred n-heptane),
◊ aromatic hydrocarbons (preferred: aromatic hydrocarbons having 5 - 10 carbon atoms; more preferred toluene),
◊ esters (preferred: C₁₋₅-alkyl esters of linear C₂₋₅ carboxylic acids; more preferred: linear C₂₋₃-alkyl esters of linear C₂₋₃ carboxylic acids; most preferred: ethyl acetate),
◊ ethers (preferred: linear, branched or cyclic C₄₋₁₀ ethers; more preferred: tetrahydrofuran) and
◊ alcohols (preferred: linear or branched C₂₋₅ alkanols; more preferred: 2-propanol), and any mixtures thereof;
more preferably the organic solvent is selected from the group consisting of aliphatic hydrocarbons, alcohols and any mixtures thereof with the preferences as given above.

It is preferred that solvents are used which are liquid under normal conditions, which allows an easy handling, such as e.g. n-heptane or 2-propanol or any mixture thereof, whereby 2-propanol is especially preferred.

The amount of the solvent is preferably chosen in such a way so that the solvent forms by weight 20 to 98% of the total solution to be hydrogenated, more preferably wherein the amount of the solvent is chosen in such a way so that the solvent forms by weight 50 to 95% of the total solution to be hydrogenated, even more preferably wherein the amount of the solvent is chosen in such a way so that the solvent forms by weight 70 to 90% of the total solution to be hydrogenated.

### Hydrogen

Usually the reduction with hydrogen (the hydrogenation) is carried out by using H₂-gas.

It is possible (and preferred) to use pure H₂-gas, but it would also be possible to use a gas mixture which comprises H₂.

Therefore the present invention relates to a process as described above wherein the hydrogenation is carried out by using (pure) H₂-gas.

### Reaction conditions

The hydrogenation is usually carried out at an absolute hydrogen pressure in the range of 1 to 20 bar, more preferably at an absolute hydrogen pressure in the range of 2 to 11 bar, even more preferably at a hydrogen pressure in the range of 4 to 9 bar, most preferably at an absolute hydrogen pressure of ca. 6 bar.

The hydrogenation is usually carried out in a vessel, which is suitable to enduring the pressure.

The hydrogenation is preferably carried out at a temperature in the range of 10 to 100°C, more preferably at a temperature in the range of 20 to 80°C, even more preferably at a temperature in the range of 40 to 60°C, most preferably at a temperature in the range of 45 to 55°C.

### Catalyst comprising nickel

The hydrogenation is carried out in the presence of a catalyst comprising nickel. US2008/177100 discloses reduction of geranonitrile in the presence of Nickel Raney. The use of an additive in not described therein.

The catalyst comprising nickel used in the process according to the present invention is a heterogeneous catalyst.

Preferred catalysts are nickel-alloy type catalysts. Such catalysts are also known as "skeletal catalysts" or "sponge-metal catalysts".

Such catalysts are commercially available for example under the trade name Actimet® from BASF (i.e. Actimet M) or under the product name B 111W, BLM 112 W, B 113 W, B 113 Z from Evonik or JM A4000, JM A40A9, JM A2000 from Johnson Matthey Catalysts or Acticat® from CatAlloy (i.e. Acticat®1000, Acticat®1100, Acticat®1200, Acticat®1600).

Preferably the catalyst comprises an amount of nickel in the range of 75 to 95% by weight and an amount of aluminium in the range of 0 to 15% by weight, with the remainder made up by other metals such that the total weight sums to 100%. These other metals are e.g. iron, chromium and/or molybdenum.

In a further preferred embodiment of the present invention, the catalyst is promoted with iron or chromium or both, preferably in an amount of 0 to 10% by weight, based on the total weight of the catalyst.

The catalyst can be re-used multiple times for further hydrogenation reactions (see table 18 and examples 8 where a re-use of four times was demonstrated) and the catalyst can also be easily recycled.
Usually the catalyst can be used without further treatment. So it is possible to run the hydrogenation batch-wise or continuously.

Preferably the amount of the catalyst is in the range of 1 to 100 weight-%, more preferably the amount of the catalyst is in the range of 10 to 80 weight-%, even more preferably the amount of the catalyst is in the range of 30 to 60 weight-%, based on the amount of the compound of formula (I).

### Additive

Suitable additives are selected preferably from the group of additives containing a sulfur atom.

Preferably the amount of the additive is in the range of 0.01 to 100 weight-%, more preferably the amount of the additive is in the range of 0.1 to 10 weight-%, even more preferably the amount of the additive is in the range of 0.2 to 2 weight-%, based on the amount of the catalyst.

### Additives containing a sulfur atom

Preferred additives containing a sulfur atom are aliphatic symmetrical disulfides, aromatic symmetrical disulfides such as diphenyldisulfide, aliphatic symmetrical sulfides, aromatic symmetrical sulfides such as diphenylsulfide, and heteroaromatic sulfides such as thiophene.

Examples of aliphatic symmetrical disulfides are disulfides R-S-S-R with R being straight chain C₂₋₆ alkyl. Examples of aliphatic symmetrical sulfides are sulfides R-S-R with R being straight chain C₂₋₆ alkyl, i.e. R being ethyl, n-propyl, n-butyl, n-pentyl and n-hexyl.

The most preferred additives containing a sulfur atom are diethyl disulfide (H₃C-CH₂-S-S-CH₂-CH₃), diphenyl disulfide, diethyl sulfide, diphenyl sulfide, diethylendisulfide, thiophene and 2,2'-(ethylenedithio)diethanol (HO-CH₂-CH₂-S-CH₂-CH₂-S-CH₂-CH₂-OH), whereby diethyl disulfide, diethyl sulfide, diphenyl sulfide, thiophene and 2,2'-(ethylenedithio)diethanol are especially preferred. The most preferred additives of this list are thiophene and 2,2'-(ethylenedithio)diethanol.

### Additives containing a nitrogen atom

Examples of amidines R-C(=NR³)-NR¹R² are ones wherein R is C₁₋₆ alkyl and R¹, R² and R³ are independently from each other hydrogen or straight chain C₁₋₆ alkyl. Additionally the alkyl groups may be linked together to form a ring.

Preferably the additive containing a nitrogen atom is chosen among C6-C10 bicyclic aliphatic amidines, C2-C8 aliphatic primary amines and C2-C6 aliphatic primary diamines.

An example of a C6-C10 bicyclic aliphatic amidine is 1,8-diaza-bicyclo[5.4.0]undec-7-ene (= "DBU").

The most preferred additives containing a nitrogen atom are 1,8-diaza-bicyclo[5.4.0]undec-7-ene ("DBU"), ethylene diamine, 1,4-diaminobutane and triethylamine, whereby 1,8-diaza-bicyclo[5.4.0]undec-7-ene, ethylene diamine and 1,4-diaminobutane are especially preferred.

### Description of the drawings

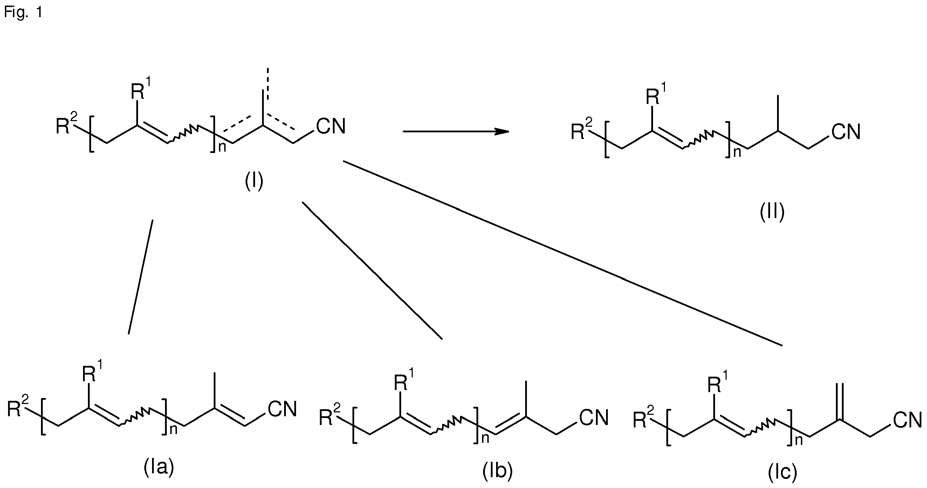

Fig. 1 illustrates the reaction of compounds of formula (I) to compounds of formula (II) according to the present invention showing the C=C double bond isomers as concrete compounds Ia, Ib and Ic;

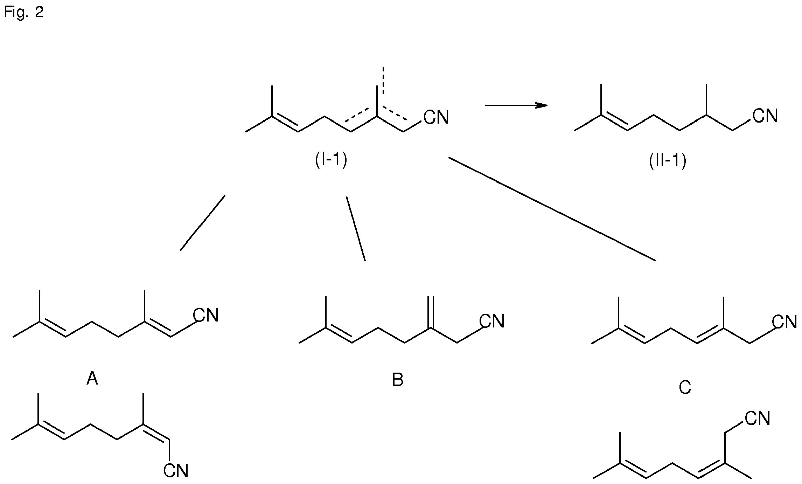

Fig. 2 illustrates the hydrogenation of a mixture of geranyl nitrile isomers (compound of formula I-1), i.e. of a mixture of (*E*/*Z*)-3,7-dimethyl-2,6-octadiene nitrile (compound A), 7-methyl-3-methylene-6-octene nitrile (compound B) and (*E*/*Z*)-3,7-dimethyl-3,6-octadiene nitrile (compound C) to citronellyl nitrile (compound of formula II-1);

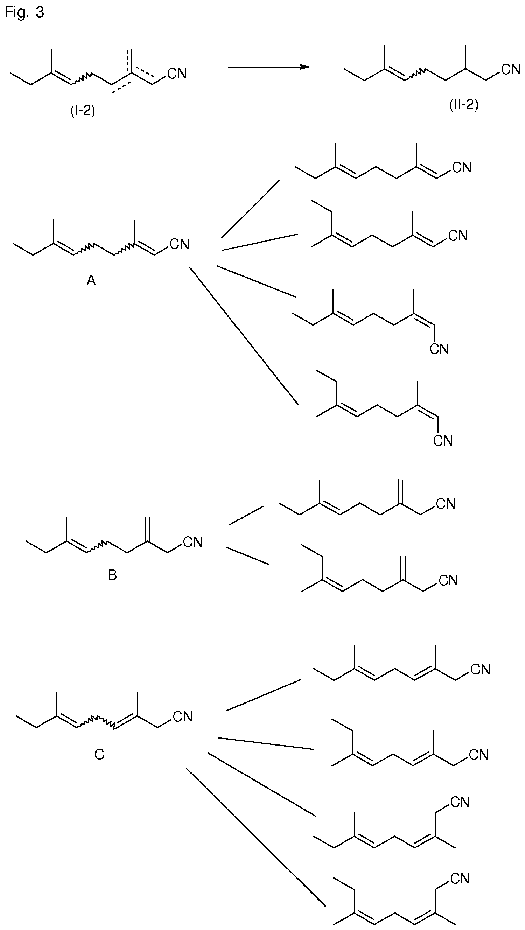

Fig. 3 illustrates the hydrogenation of methyl limonitrile (compound of formula I-2); i.e. a mixture of 3,7-dimethyl-2,6-nonadiene nitrile (compound A), 7-methyl-3-methylene-6-nonene nitrile (compound B) and 3,7-dimethyl-3,6-nonadiene nitrile (compound C) to (*E*/*Z*)-3,7-dimethyl-6-nonennitrile (compound of formula II-2).

Besides a mixture of geranyl nitrile isomers of formula I-1 it is also possible to hydrogenate any other mixture of geranyl nitrile ((E)-3,7-dimethyl-2,6-octadiene nitrile) and neryl nitrile ((Z)-3,7-dimethyl-2,6-octadiene nitrile) or the single compounds according to the process of the present invention to obtain citronellyl nitrile. Since geranyl nitrile is toxic there is an increasing demand of substituting geranyl nitrile by another fragrance such as a compound of formula (II), which does not have the toxicological disadvantages of geranyl nitrile.

### Novel compounds

Since the compounds of formula (II) - with the proviso that R² is not hydrogen if R¹ = methyl and n = 1 or 2 - are novel, the present invention is also directed to them. Thus, a further embodiment of the present invention is a compound of formula (II) 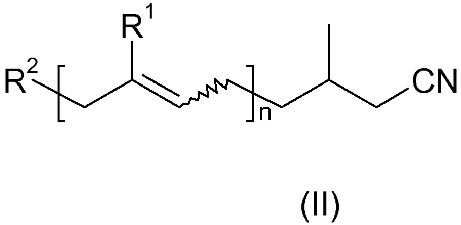 wherein n is either 1 or 2, preferably wherein n = 1;
wherein R¹ is linear C₁₋₄ alkyl or branched C₃₋₄ alkyl,
preferably wherein R¹ is methyl or ethyl, and
wherein R² is hydrogen or linear C₁₋₄ alkyl or branched C₃₋₄ alkyl,
preferably wherein R² is hydrogen or methyl,
with the proviso that R² is not hydrogen if R¹ = methyl and n = 1 or 2.

### Novel use

Furthermore, the invention also relates to the use of a compound of formula (II) with the proviso that R² is not hydrogen if R¹ = methyl and n = 1, especially of a compound of formula II-2, as aroma ingredient.

In the flavor and fragrance industry there is always a need and demand for compounds that enhance, modify, improve or otherwise positively influence an odor note and therefore give perfumers or other persons the ability to create new fragrances for perfumes, colognes, personal care products, household products or any other products, which comprise aroma ingredients, i.e. well-defined substances with characteristic aroma. Flavors and fragrances are compositions made of several aroma ingredients.

Surprisingly it was found that the compounds of formula (II), with the proviso that R² is not hydrogen if R¹ = methyl and n = 1, especially the compound of formula II-2, are very useful in flavor and fragrance compositions.
Therefore the present invention is related to the use of a compound of formula (II) with the proviso that R² is not hydrogen if R¹ = methyl and n = 1, especially of a compound of formula II-2, in flavor and/or fragrance applications.
The compounds of formula (II) with the proviso that R² is not hydrogen if R¹ = methyl and n = 1, especially the compound of formula II-2, may be used as such or in combination with other compounds of formula (II) or other compounds which are known as aroma ingredients.

Such other compounds which are known as aroma ingredients include all known odorant molecules selected from the extensive range of natural products and synthetic molecules currently available, such as essential oils, alcohols, aldehydes and ketones, ethers and acetals, esters and lactones, macrocycles and heterocycles, and/or mixed with one or more ingredients or excipients conventionally used in conjunction with odorants in flavor or fragrance formulations, for example, carrier materials, and other auxiliary agents commonly used in the art.

The aroma ingredients of the present invention, i.e. the compounds of formula II, are used in a flavor and fragrance formulation.
Such a flavor and fragrance formulation comprises other ingredients.
The flavor and fragrance formulation according to the present invention can be in any form. Usually it is in a solid, gel-like or liquid (or a combination thereof) form. It can also be in an encapsulated form (i.e. a liquid formulation encapsulated by a suitable matrix material).

Therefore the present invention also relates to flavor and fragrance formulations comprising
(i) at least one compound of formula (II) with the proviso that R² is not hydrogen if R¹ = methyl and n = 1, especially the compound of formula II-2.

When a compound of formula (II) is used in a flavor and fragrance formulation, then the amount thereof is in the range of 0.0001 - 10 weight-% (wt-%), related to the total weight of the flavor and fragrance formulation. Preferably is an amount in the range of 0.01 - 5 wt-%, based on the total weight of the flavor and fragrance formulation.

Therefore the present invention relates to liquid flavor and fragrance formulations comprising
(i) 0.0001 - 10 wt-% (preferably 0.01 - 5 wt-%), related to the total weight of the flavor and fragrance formulation, of at least one compound of formula (II) with the proviso that R² is not hydrogen if R¹ = methyl and n = 1, especially of a compound of formula II-2.

The flavor and fragrance formulations according to the present invention can comprise further ingredients (= auxiliary compounds), such as any further perfuming compounds, solvents, adjuvants, thickeners, surface active agents, pigments, extenders, rheology modifiers, dyestuffs, antioxidants, fillers and the like.

Many flavor and fragrance formulations are in a liquid form (like a perfume, cologne, etc.). Therefore, for such liquid formulation a (diluent) solvent is present. Such common diluents are i.e. dipropyleneglycol, isopropyl myristate, triethylcitrate and alcohols (such as ethanol).

Further examples of fine perfumery are Eau de perfume, Eau de Toilette, Eau de Cologne and Splash Cologne. Fine perfumery products are commonly based on an alcoholic solution as diluent. However fine perfumery products using an oil or wax as diluent are also included within the meaning of this invention. The compounds can be employed in widely varying amounts, depending upon the specific application and on the nature and quantity of other odourant ingredients.

When used in a (fine) perfume, the amount of the compound of formula (II) with the proviso that R² is not hydrogen if R¹ = methyl and n = 1, especially of a compound of formula II-2, is usually between 0.01 - 10 wt-%, based on the total weight of the (fine) perfume.
However, these values and ranges are given only by way of example, since the experienced perfumer may also achieve effects or may create novel accords with lower or higher concentrations.

Furthermore the present invention relates to liquid flavor and fragrance formulations comprising
(i) at least one compound of formula (II) with the proviso that R² is not hydrogen if R¹ = methyl and n = 1, especially the compound of formula II-2, and
(ii) at least one diluent chosen from the group consisting of dipropyleneglycol, isopropylmyristate, triethylcitrate and alcohols (such as ethanol), and optionally
(iii) at least one auxiliary compound selected from the group consisting of perfuming compounds, solvents, adjuvants, thickeners, surface active agents, pigments, extenders, rheology modifiers, dyestuffs, antioxidants and fillers.

Furthermore the present invention relates to solid flavor and fragrance formulations comprising
(i) at least one compound of formula (II) with the proviso that R² is not hydrogen if R¹ = methyl and n = 1, especially the compound of formula II-2, and
(ii) at least one auxiliary compound selected from the group consisting of perfuming compounds, solvents, adjuvants, thickeners, surface active agents, pigments, extenders, rheology modifiers, dyestuffs, antioxidants and fillers.

The compounds of formula (II) with the proviso that R² is not hydrogen if R¹ = methyl and n = 1, especially the compound of formula II-2, may be used in a broad range of flavor and fragrance formulations, e.g. in any field of fine and functional perfumery, such as perfumes, air care products, household products, laundry products, body care products and cosmetics.

The compounds as described hereinabove may be employed in a flavor and fragrance formulation simply by directly mixing at least one compound of formula (II), a mixture thereof, or a fragrance composition with the other ingredients used in the final product, or they may, in an earlier step, be entrapped with an entrapment material, for example, polymers, capsules, microcapsules and nanocapsules, liposomes, film formers, absorbents such as carbon or zeolites, cyclic oligosaccharides and mixtures thereof, or they may be chemically bonded to substrates, which are adapted to release the fragrance molecule upon application of an external stimulus such as light, enzyme, or the like, and then mixed with the other ingredients used in the final product.

Thus, the invention additionally provides a method of manufacturing a flavor and fragrance formulation, comprising the incorporation of a compound of formula (II) with the proviso that R² is not hydrogen if R¹ = methyl and n = 1, especially of the compound of formula II-2, as a fragrance ingredient, either by directly admixing the compound to the other ingredients used in the final product or by admixing a fragrance composition comprising a compound of formula (I), which may then be mixed with the other ingredients used in the final product, using conventional techniques and methods.

Through the addition of an olfactory acceptable amount of a compound of the present invention as hereinabove described, or a mixture thereof, the odor notes of a consumer product base will be improved, enhanced or modified.

Thus, the invention furthermore provides a method for improving, enhancing or modifying a flavor or fragrance formulation as well as a consumer product (= final product) base by means of the addition thereto of an olfactory acceptable amount of a compound of formula (II) with the proviso that R² is not hydrogen if R¹ = methyl and n = 1, especially of the compound of formula II-2, or a mixture thereof.

In the context of the present invention the olfactory effective amount is to be understood as the amount of the at least one compound of formula (II) with the proviso that R² is not hydrogen if R¹ = methyl and n = 1, especially of the compound of formula II-2, in a flavor and fragrance formulation which will contribute to its particular olfactory characteristics, but the olfactory effect of the flavor and fragrance formulation will be the sum of the effects of each of the perfumes or fragrance ingredients. Thus the compounds of the invention can be used to alter the aroma characteristics of the flavor and fragrance formulation, or by modifying the olfactory reaction contributed by another ingredient in the composition. The amount will vary depending on many factors including other ingredients, their relative amounts and the effect that is desired.

As used herein, "consumer product (= final product)" means a composition for use as a consumer product to fulfill specific actions, such as cleaning, softening, and caring or the like. Examples of such products include fine perfumery, e.g. perfume and eau de toilette; fabric care, household products and personal care products such as laundry care detergents, rinse conditioner, personal cleansing composition, detergent for dishwasher, surface cleaner; laundry products, e.g. softener, bleach, detergent; body care products, e.g. shampoo, shower gel; air care products and cosmetics, e.g. deodorant, vanishing creme. This list of products is given by way of illustration and is not to be regarded as being in any way limiting.

The present invention is also directed to a process for the manufacture of a mixture of geranyl nitril isomers, i.e. of a mixture of (*E*/*Z*)-3,7-dimethyl-2,6-octadiene nitrile (compound A), 7-methyl-3-methylene-6-octene nitrile (compound B) and (*E*/*Z*)-3,7-dimethyl-3,6-octadiene nitrile (compound C).

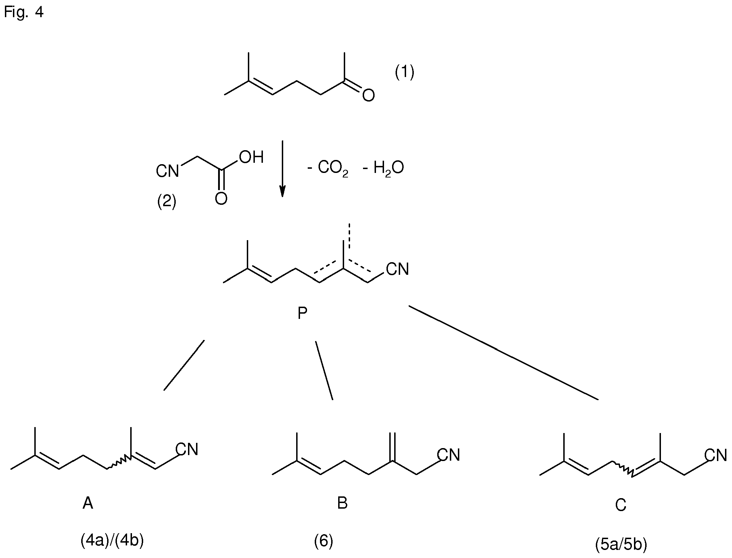

This process according to the invention comprises the step of reacting 6-methyl-5-hepten-2-one (MH) with cyanoacetic acid and removing water and carbon dioxide as shown in Fig. 4, wherein the reaction and the removal of water and carbon dioxide are performed in the presence of a base and a co-base 1 and in an organic solvent.

Geranyl nitrile ((E)-3,7-dimethyl-2,6-octadiene nitrile) and neryl nitrile ((Z)-3,7-dimethyl-2,6-octadiene nitrile) are commonly known aroma ingredients, i.e. well-defined substances with characteristic aroma which are used in processed food and beverages, or in consumer goods such as perfumes, toiletries, cosmetics, detergents and household cleaners. Geranyl nitrile and neryl nitrile are used in flavours and fragrances which are compositions made of several aroma ingredients. Furthermore, they are intermediates for other aroma ingredients.

There is still a need for a process to manufacture geranyl nitrile isomers A, B and C in an efficient and economic way at industrial scale as starting material for citronellyl nitrile.

### Detailed description

This need is fulfilled by the present invention, which is directed to a process for the manufacture of a mixture of (*E*/*Z*)-3,7-dimethyl-2,6-octadiene nitrile, (*E*/*Z*)-3,7-dimethyl-3,6-octadiene nitrile and 7-methyl-3-methylene-6-octene nitrile comprising the following steps:
a) reacting 6-methyl-5-hepten-2-one with cyanoacetic acid and removing carbon dioxide and water, wherein the reaction and the removal of carbon dioxide and water are performed in the presence of a base and a co-base 1 in an organic solvent, wherein the base is preferably pyridine, and
   wherein the organic solvent is a solvent which forms a heteroazeotrop with water;
b) removing the solvent and the base (preferably pyridine) of the reaction mixture obtained after having performed step a) by distillation to obtain a mixture of (*E*/*Z*)-3,7-dimethyl-2,6-octadiene nitrile, (*E*/*Z*)-3,7-dimethyl-3,6-octadiene nitrile and 7-methyl-3-methylene-6-octene nitrile.

The process of the present invention may comprise an isomerization step c), whereby step b) can be performed before or after step c).

This additional step is:
c) isomerizing the mixture obtained after having performed step a) or step b) by heating this mixture to a temperature in the range of 80 to 160°C, preferably to a temperature in the range of 90 to 150°C, more preferably to a temperature in the range of 100 to 140°C, in the presence of a co-base 2 to obtain an isomerized reaction mixture.

Thus, if the isomerization step c) is performed after step b) the process is as follows:
A process for the manufacture of a mixture of (*E*/*Z*)-3,7-dimethyl-2,6-octadiene nitrile, (*E*/*Z*)-3,7-dimethyl-3,6-octadiene nitrile and 7-methyl-3-methylene-6-octene nitrile comprising the following steps:
a) reacting 6-methyl-5-hepten-2-one with cyanoacetic acid and removing carbon dioxide and water, wherein the reaction and the removal of carbon dioxide and water are performed in the presence of a base and a co-base 1 in an organic solvent,
   wherein the base is preferably pyridine, and
   wherein the organic solvent is a solvent which forms a heteroazeotrop with water;
b) removing the solvent and the base (preferably pyridine) of the reaction mixture obtained after having performed step a) by distillation to obtain a mixture of (*E*/*Z*)-3,7-dimethyl-2,6-octadiene nitrile, (*E*/*Z*)-3,7-dimethyl-3,6-octadiene nitrile and 7-methyl-3-methylene-6-octene nitrile;
c) isomerizing the mixture obtained after having performed step b) by heating this mixture to a temperature in the range of 80 to 160°C, preferably to a temperature in the range of 90 to 150°C, more preferably to a temperature in the range of 100 to 140°C, in the presence of a co-base 2 to obtain an isomerized reaction mixture.

Accordingly, if step b) is performed after the isomerization step c) the process is as follows:
A process for the manufacture of a mixture of (*E*/*Z*)-3,7-dimethyl-2,6-octadiene nitrile, (*E*/*Z*)-3,7-dimethyl-3,6-octadiene nitrile and 7-methyl-3-methylene-6-octene nitrile comprising the following steps:
a) reacting 6-methyl-5-hepten-2-one with cyanoacetic acid and removing carbon dioxide and water, wherein the reaction and the removal of carbon dioxide and water are performed in the presence of a base and a co-base 1 in an organic solvent,
   wherein the base is preferably pyridine, and
   wherein the organic solvent is a solvent which forms a heteroazeotrop with water;
c) isomerizing the mixture obtained after having performed step a) by heating this mixture to a temperature in the range of 80 to 160°C, preferably to a temperature in the range of 90 to 150°C, more preferably to a temperature in the range of 100 to 140°C, in the presence of a co-base 2 to obtain an isomerized reaction mixture;
b) removing the solvent and the base (preferably pyridine) of the isomerized reaction mixture obtained after having performed step c) by distillation to obtain a mixture of (*E*/*Z*)-3,7-dimethyl-2,6-octadiene nitrile, (*E*/*Z*)-3,7-dimethyl-3,6-octadiene nitrile and 7-methyl-3-methylene-6-octene nitrile.

The co-base 2 used in step c) can be the co-base 1 still present in the reaction mixture obtained from step a).

### Starting materials:

Cyanoacetic acid (**2**) and 6-methyl-5-hepten-2-one (MH, **1**) are used as starting materials. Usually the molar ratio of cyanoacetic acid to MH is in the range of (0.8 to 1.5 mol) : 1 mol, preferably in the range of (0.9 to 1.3 mol) : 1 mol, more preferably in the range of (1.00 to 1.25 mol) : 1 mol, most preferably in the range of (1.00 to 1.15 mol) : 1 mol.

### Base

Preferably pyridine is used as base; suitable other bases are 2-methyl pyridine, 3-methyl pyridine, 2-ethyl pyridine, 3-ethyl-pyridine, 2,3-lutidine (2,3-dimethyl pyridine), 2,4-lutidine (2,4-dimethyl pyridine), 2,5-lutidine (2,5-dimethyl pyridine), 2,6-lutidine (2,6-dimethyl pyridine), 2,3,4-collidine (2,3,4-trimethyl pyridine), 2,3,5-collidine (2,3,5-trimethyl pyridine), 2,3,6-collidine (2,3,6-trimethyl pyridine), 2,4,6-collidine (2,4,6-trimethyl pyridine), and piperidine, as well as any mixture thereof.

Preferably a single base is used. Of the bases named pyridine, 2-methyl pyridine, 3-methyl pyridine and piperidine are preferred. More preferred examples are pyridine, 2-methyl pyridine and piperidine. The most preferred base is pyridine.

Preferably the amount of the base is in the range of 0.4 to 1.5 mol per mol of MH, more preferably the amount of the base is in the range of 0.5 to 1.25 mol per mol of MH. These amounts especially also apply when pyridine is used as base.

### Co-bases 1 and 2

The co-bases 1 and 2 are preferably independently selected from each other from the group consisting of 1,4-diamino butane, 1,5-diaminopentane, piperidine, morpholine, ethylendiamine, diethylentriamine, ammonium chloride and ammonium acetate as well as any mixture thereof.

Preferably a single co-base 1 and a single co-base 2 are used.

More preferably the co-base 1 and the co-base 2 are independently from each other either 1,4-diaminobutane or ammonium acetate.

Most preferably 1,4-diaminobutane ("DAB") is used as co-base 1 and as co-base 2. DAB is an intermediate produced by DSM as well as by other suppliers.

Preferably the amount of the co-base 1 in step a) is in the range of 0.005 to 0.15 mol per mol of MH, more preferably the amount of the co-base 1 is in the range of 0.01 to 0.1 mol per mol of MH, most preferably the amount of the co-base 1 is in the range of 0.01 to 0.05 mol per mol of MH.

Preferably the amount of the co-base 2 in step c) is in the range of 0.005 to 0.15 mol per mol of MH, more preferably the amount of the co-base 2 is in the range of 0.01 to 0.1 mol per mol of MH, most preferably the amount of the co-base 2 is in the range of 0.01 to 0.05 mol per mol of MH.

The co-base 1 and 2, respectively, may be used as such, i.e. in substance, or also as solution, e.g. as aqueous solution. This is especially advantageous for 1,4-diaminobutane which has a melting point of 27-28°C at atmospheric pressure, i.e. it is solid at room temperature, since a-solution of DAB facilitates its dosing. The concentration of such solution is not critical. The same applies for ammonium acetate which may also be used as solution.

### Solvent

Forming a heteroazeotrop with water means that solvent and water form an azeotrop whereby solvent and water are not or only partially miscible with each other.

Preferred examples of such solvents are methoxypentan, 2-methyltetrahydrofuran, ortho-xylene, meta-xylene, para-xylene, hexane, cyclohexane, n-heptane, toluene, and any mixture thereof. More preferred only one solvent of this group is used and not a mixture of two or more solvents. Even more preferred n-heptane or toluene is used as solvent. Most preferred n-heptane is used as solvent.

The amount of solvent is preferably chosen in a way that the amount of MH is in the range of 0.5 to 8 mol per 1 liter of solvent, more preferably that the amount of MH is in the range of 1.0 to 6.0 mol per 1 liter of solvent, even more preferably that the amount of MH is in the range of 2.0 to 4.5 mol per 1 liter of solvent, most preferably that the amount of MH is in the range of 3.0 to 4.0 mol per 1 liter of solvent. These preferred amounts especially also apply if n-heptane or toluene is used as solvent.

### Without solvent (see examples 36 and 37)

Since the base pyridine also forms an azeotrop with water, it is also possible to perform the condensation of MH with cyanoacetic acid to the mixture of the geranyl nitril isomers without an additional organic solvent. Then in step b) only the base (preferably pyridine) is removed.

Thus, the present invention is also directed to a process for the manufacture of a mixture of (*E*/*Z*)-3,7-dimethyl-2,6-octadiene nitrile, (*E*/*Z*)-3,7-dimethyl-3,6-octadiene nitrile and 7-methyl-3-methylene-6-octene nitrile comprising the following steps:
a) reacting 6-methyl-5-hepten-2-one with cyanoacetic acid and removing carbon dioxide and water, wherein the reaction and the removal of carbon dioxide and water are performed in the presence of a base and a co-base 1, wherein the base is preferably pyridine, and
b) removing the base of the reaction mixture obtained after having performed step a) by distillation to obtain a mixture of (*E*/*Z*)-3,7-dimethyl-2,6-octadiene nitrile, (*E*/*Z*)-3,7-dimethyl-3,6-octadiene nitrile and 7-methyl-3-methylene-6-octene nitrile.

Also in this case an additional isomerization step as defined above may be performed after step a) or after step b).

### Reaction conditions

The manufacture of the mixture of geranyl nitrile isomers can be divided in several steps which are described in more detail below.

These steps are:
a) reaction of cyanoacetic acid and MH in presence of a base and a co-base 1;
b) removal of the solvent (if present) and the base by distillation;
   if no solvent is present: removal of the base by distillation;
c) optional isomerization of the reaction mixture (whereby the co-base 1 is still present or a co-base 2 is added) obtained in step b) to a desired isomer ratio by heating the reaction mixture to a temperature in the range of 80 to 160°C;
d) optional dilution of the mixture obtained in step b) or the isomerized mixture in step c) with the solvent; extracting the mixture with diluted acids, whereby an organic phase and an aqueous phase are formed; separating the organic phase containing the mixture of the geranyl nitril isomers from the aqueous phase; washing the organic phase one or more times with deionized water, and optionally also with aqueous bases; removing the solvent, if present, from the organic phase resulting in a purified mixture of geranyl nitril isomers;
e) distillation of the mixture;
f) recycling of non-reacted 6-methyl-5-hepten-2-one (MH) completely or partially back to the reaction step a). (Step f) is only possible if step e) has been carried out.)

Steps c) to f) are independently from each other optional. Preferably steps d) and e) are carried out.

It is also possible to carry out step c) before step b), i.e. to carry out the process with the order of the steps as follows: a), c), b). In this case the performance of step b) would also result in an "isomerized" mixture of (E/Z)-3,7-dimethyl-2,6-octadiene nitrile, 7-methyl-3-methylene-6-octene nitrile and (E/Z)-3,7-dimethyl-3,6-octadiene nitrile.
Depending on which of these steps are performed different qualities of the mixture of **4a, 4b, 5a, 5b** and **6** are obtained.

In the most preferred embodiment of the present invention all preferred conditions for each step a) to f) are realized; pyridine is used as base with the preferred amounts as given above, 1,4-diamino butane is used as co-base with the preferred amounts as given above, n-heptane is used as solvent with the preferred amounts as given above, and cyanoacetic acid and MH are also used with the preferred amounts as given above.

All steps a) to f) are preferably carried out at inert conditions.
A further advantage of the present invention is that steps a) to c) can be carried out as a one-pot-process.

### Step a) Condensation

The condensation is carried out at a pressure in the range of from 0.3 to 1.5 bar (absolute pressure), preferably at a pressure in the range of from 0.5 to 1.2 bar (absolute pressure), more preferably at a pressure in the range of from 0.7 to 1.0 bar (absolute pressure). The temperature at which the reaction of MH and cyanoacetic acid and the removal of H₂O and CO₂ is performed is defined by the temperature at which the reaction mixture is under reflux.

If n-heptane is used as solvent, the temperature is preferably in the range of 105 to 112°C; if toluene is used as solvent, the temperature is preferably in the range of 100 to 112°C (see **table 3**).

Advantageously the condensation is carried out under an argon flow or nitrogen flow, preferably an argon flow or nitrogen flow in the range of 15 to 40 liter per hour, more preferably an argon flow in the range of 15 to 40 liter per hour.

### Step b) Removal of the solvent and the base by distillation

This step is carried out at a final temperature in the range of from 100 to 180°C, preferably at a final temperature in the range of from 120 to 160°C, more preferably at a final temperature in the range of from 130 to 150°C.

In case n-heptane is the solvent and pyridine is the base this step is carried out most preferably at a final temperature in the range of from 100 to 110°C. The distilled off solvent mixture (solvent and base; especially n-heptane and pyridine) can be used for further reaction batches within the manufacture of the isomers of geranyl nitrile, i.e. it can be recycled back into step a).

The pressure at which this step is performed is preferably in the range of from 0.05 to 1 bar (absolute pressure), preferably in the range of from 0.1 to 1 bar (absolute pressure).

It is recommended to almost completely remove the solvent and the base, as well as to recycle them back in step a).

### Step c) Isomerization of the reaction mixture obtained in step a) or step b)

This step is carried out at a temperature in the range of from 80 to 160°C, preferably at a temperature in the range of from 90 to 150°C, more preferably at a temperature in the range of from 100 to 140°C. The pressure at which the isomerization is performed is not relevant and for practical reasons chosen within a range of from 0.1 to 2 bar (absolute pressure). Most convenient this step is performed at atmospheric pressure.

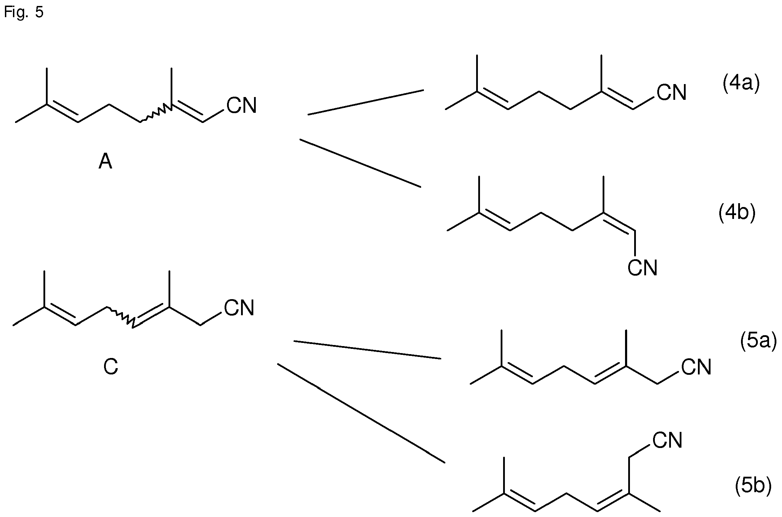

Though the solvent and the base have been removed in step b), it can be that the co-base 1 is still present. If not, a co-base 2 which can be the same as co-base 1 or a different one can be added. Thus, the co-base 1 and/or the co-base 2 is the catalyst for the isomerization beside being the catalyst for the formation of the 5 stereoisomers (**4a, 4b, 5a, 5b, 6**) as shown in **Fig. 4** and **5****.**

### Step d) Purification of the reaction mixture

For the extraction of the mixture of compounds A, B and C from the raw product obtained in step c), in case step c) is performed after step b), or from the raw product obtained in step b), in case step b) is performed after step c) or in case step c) is not performed at all, preferably an aqueous solution of a strong acid is used as extraction medium after the mixture has been preferably diluted with an organic solvent (preferably with an organic solvent selected from the group as described above, more preferably with n-heptane).

Examples of such extraction media are aqueous solutions of sulfuric acid, hydrochloric acid and methane sulfonic acid, whereby aqueous solutions of sulfuric acid are preferred. More preferred the extraction media have the following concentrations:
Diluted aqueous sulfuric acid: 1-10 weight-%, more preferred 4-8 weight-%;
diluted aqueous hydrochloric acid: 1-10 weight-%, more preferred 4-8 weight-%;
diluted aqueous methane sulfonic acid: 5-20 weight-%, more preferred 8-15 weight-%, whereby diluted aqueous sulfuric acid with the concentrations as given above is most preferred.

When the extraction medium is added to the raw product obtained in step c)/b), optionally diluted with the organic solvent, two phases are formed: an organic phase and an aqueous phase.

The treatment of the crude product obtained in step c)/b) with the extraction medium is preferably carried out at a temperature in the range of from 5 to 25°C, more preferably at a temperature in the range of from 5 to 20°C, most preferably at a temperature in the range of from 5 to 15°C.

The organic phase containing the mixture of the isomers **4a, 4b, 5a, 5b** and **6** is separated from the aqueous phase and washed with deionised water. The washing with deionised water can be carried out several times.

Optionally the organic phase can also be washed with aqueous bases. Examples of such aqueous bases are sodium hydrogencarbonate solutions and aqueous sodium carbonate solutions. Preferably such aqueous bases have a concentration of 1 mol/l.

The removal of the solvent, especially of n-heptane, may be achieved by rectification or distillation and can be carried out batch-wise or continuously. During the removal of the solvent, especially of the n-heptane, small amounts of water and organic base are also removed.

The aqueous phases, i.e. the aqueous phase as obtained when the organic phase is separated off and the combined de-ionised wash waters, can also be back-extracted with the solvent, especially with n-heptane, whereby a further organic phase is obtained which contains small amounts of water. The back-extraction increases the yield of the mixture of isomers **4a, 4b, 5a, 5b** and **6** but has the disadvantage in having to remove a bigger amount of solvent. Thus, preferably the back-extraction is **not** carried out. If it is, however, carried out, the thus removed n-heptane is advantageously recycled back into this step and used again for further back-extraction.

### Step e)

Since steps c) to d) are independently from each other optional,
either the reaction mixture obtained in step b) (if step c) is not performed or if step c) is performed before step b)) or
the isomerized reaction mixture obtained in step c) (if step c) is performed after step b)) or the purified reaction mixture (if step c) is not performed, but step d)) or
the purified isomerized reaction mixture (if steps c) and d) are performed) are distilled to obtain preferably a colourless liquid mixture of isomers **4a, 4b, 5a, 5b** and **6.**

If step b) is performed after step c) also an isomerized reaction mixture is obtained which can then be distilled.

Furthermore, it is also possible to separate the starting material, i.e. non-reacted MH.

### Step f) Recycling of non-reacted 6-methyl-5-hepten-2-one (MH)

The non-reacted MH obtained back in step e) is preferably completely (preferred) or partially taken back to the reaction (step a)) with cyanoacetic acid in presence of a solvent (preferred n-heptane), a base (pyridine) and a co-base (preferably DAB) to partially substitute "fresh" MH. To avoid accumulation of by-products it might be advantageous to purge a part of this recycle stream from time to time.

### Product

The reaction mixture consists of 5 isomers (compounds A, B and C), wherein 3,7-dimethyl-2,6-octadiene nitrile (compound A) is in fact a mixture of two geometrical isomers as shown in **Fig. 5****:** (*E*)-3,7-dimethyl-2,6-octadiene nitrile and (*Z*)-3,7-dimethyl-2,6-octadiene nitrile.

3,7-Dimethyl-3,6-octadiene nitrile (compound C) is also a mixture of two isomers (see **Fig. 5**): (*E*)-3,7-dimethyl-3,6-octadiene nitrile and (*Z*)-3,7-dimethyl-3,6-octadiene nitrile.

### Description of the drawings

**Fig. 4** shows the manufacture of the mixture of geranyl nitrile isomers (= compound P consisting in fact of compounds A, B and C and **4a, 4b, 5a, 5b** and **6,** respectively) (step a).

**Fig. 5** shows the 4 geranyl nitrile isomers (compounds A and C and **4a, 4b, 5a** and **5b,** respectively).

"A" means "compound A", "B" means "compound B", "C" means "compound C" and "P" means "compound P".

### Most preferred embodiment of the present invention

The most preferred embodiment of the present invention is a process for the manufacture of a mixture of (*E*/*Z*)-3,7-dimethyl-2,6-octadiene nitrile, (*E*/*Z*)-3,7-dimethyl-3,6-octadiene nitrile and 7-methyl-3-methylene-6-octene nitrile comprising the following steps:
i) reacting 6-methyl-5-hepten-2-one with cyanoacetic acid and removing carbon dioxide and water, wherein the reaction and the removal of carbon dioxide and water are performed in the presence of pyridine as base and 1,4-diaminobutane or ammonium acetate as co-base and in n-heptane or toluene as organic solvent;
ii) removing the organic solvent and pyridine by distillation to obtain a reaction mixture;
iii) diluting the mixture obtained in step ii) with the organic solvent; extracting the mixture with diluted acids whereby an organic phase and an aqueous phase are formed; separating the organic phase containing the mixture of the geranyl nitrile isomers from the aqueous phase; washing the organic phase one or more times with deionized water, and also with aqueous bases; removing the organic solvent from the organic phase resulting in a purified mixture of geranyl nitril isomers;
iv) distilling the organic phase obtained in step iii) to obtain a mixture of the geranyl nitril isomers and optionally non-reacted MH;
v) optionally recycling the MH obtained in step iv) back into step i).

Steps i) to v) are performed as described above for steps a), b), d), e) and f), also with the preferred conditions as given there.

### Step v)

Preferably this step is performed, i.e. the thus removed non-converted MH is completely (preferred) or partially recycled back into step a).

The invention is now further illustrated in the following non-limiting examples.

### Examples

6-Methyl-5-hepten-2-one (Aldrich M48805, 99%), cyanoacetic acid (Fluka 28400, > 99.0%), pyridine (Fluka 360570, ≥ 99.0%, 1,4-diamino-butane (Merck, ≥ 98.0%), n-heptane (Fluka 51750, ≥ 99.0%), toluene (Fluka 89681, ≥ 99.7%), ammonium acetate (Fluka 09690, ≥ 98.0%) are commercially available and were used without further purification.

### I. Condensation examples 1-58

The conversion is based on the amount of 6-methyl-5-hepten-2-one (MH, 1).

The yield is the molar amount of the isomers **4 + 5 + 6** based on the molar amount of 6-methyl-5-hepten-2-one.

The selectivity is the molar amount of the isomers **4 + 5 + 6,** based on the molar amount of converted 6-methyl-5-hepten-2-one.

### Condensation examples 1-5: Variation of the solvent

### Example 1: Preparation of a mixture of (E/Z)-3,7-dimethyl-2,6-octadiene nitrile, (E/Z)-3,7-dimethyl-3,6-octadiene nitrile and 7-methyl-3-methylene-6-octene nitrile in toluene

6.4 g (50 mmol) of 6-methyl-5-hepten-2-one (MH, **1**), 4.5 g (52.5 mmol) of cyanoacetic acid (**2**), 5 ml (62.5 mmol) of pyridine, 10 ml of toluene and 51 µl (0.5 mmol) of 1,4-diamino-butane are filled into a 50 ml glass reactor (double jacketed) with reflux condenser, dean stark water separator and stirrer. The mixture is stirred at 450 rpm (resolutions per minute) and heated to 112°C (internal temperature). The mixture is held under reflux and an Argon flow for 8 hours including water and carbon dioxide removal. The resulting mixture is cooled to room temperature (20°C) and the solvent and the base are evaporated under reduced pressure (20 mbar, 40°C). A brown liquid (6.9 g) is obtained (content: 88.0 % of a mixture of isomers **4, 5,** and **6,** analyzed by GC ISTD). The yield (**4, 5,** and **6**) was 80.8 % based on **1** (selectivity 85.7%; conversion based on **1:** 94.3%).

### Composition of the product P as analyzed by gas chromatography (GC):

(*Z*)-3,7-dimethylocta-2,6-dienenitrile (**4b**) + 7-methyl-3-methyleneoct-6-enenitrile (**6**): 12.8 % GC
(*Z*)-3,7-dimethylocta-3,6-dienenitrile (**5b**): 33.1 % GC
(*E*)-3,7-dimethylocta-2,6-dienenitrile (**4a**): 38.1 % GC
(*E*)-3,7-dimethylocta-3,6-dienenitrile (**5a**): 34.0 % GC

### Example 2: Preparation of a mixture of (E/Z)-3,7-dimethyl-2,6-octadiene nitrile, (E/Z)-3,7-dimethyl-3,6-octadiene nitrile and 7-methyl-3-methylene-6-octene nitrile in n-heptane

6.4 g (50 mmol) of 6-methyl-5-hepten-2-one (MH, **1**), 4.5 g (52.5 mmol) of cyanoacetic acid (**2**), 5 ml (62.5 mmol) of pyridine, 10 ml of n-heptane and 51 µl (0.5 mmol) of 1,4-diamino-butane are filled into a 50 ml glass reactor (double jacketed) with reflux condenser, dean stark water separator and stirrer. The mixture is stirred at 450 rpm (resolutions per minute) and heated to 112°C (reflux temperature). The mixture is held under reflux and an Argon flow for 8 hours including water and carbon dioxide removal. After cooling (to 20°C), the reaction mixture is evaporated under reduced pressure (20 mbar, 40°C). 7.5 g of a brown liquid is obtained (content: 86.0 % of a mixture of isomers **4, 5,** and **6** analyzed by GC (ISTD). The yield is 86.2% based on **1** and the selectivity is 87.4% (conversion based on **1:** 98.6%).

### Composition of the product P as analyzed by gas chromatography (GC):

(Z)-3,7-dimethylocta-2,6-dienenitrile (**4b**) + 7-methyl-3-methyleneoct-6-enenitrile (**6**): 18.9% GC
(Z)-3,7-dimethylocta-3,6-dienenitrile (**5b**): 0.4% GC
(*E*)-3,7-dimethylocta-2,6-dienenitrile (**4a**): 41.9% GC
(*E*)-3,7-dimethylocta-3,6-dienenitrile (**5a**): 24.7% GC

### Example 3: Preparation of a mixture of (E/Z)-3,7-dimethyl-2,6-octadiene nitrile, (E/Z)-3,7-dimethyl-3,6-octadiene nitrile and 7-methyl-3-methylene-6-octene nitrile in cyclohexane

In an analogous manner to examples 1 and 2 example 3 is carried out, whereby 10 ml of cyclohexane are used as solvent instead of toluene or n-heptane. The internal temperature is 92°C. The conversion based on **1** is 92.7%, the yield (**4 + 5 + 6** based on 1) is 78.7% and the selectivity of **4 + 5 + 6** is 84.9%.

### Comparison examples 4-5

### Comparison example 4: Preparation of a mixture of (E/Z)-3,7-dimethyl-2,6-octadiene nitrile, (E/Z)-3,7-dimethyl-3,6-octadiene nitrile and 7-methyl-3-methylene-6-octene nitrile in methyl tert-butyl ether

In an analogous manner to examples 1-3 example 4 is carried out, whereby 10 ml of methyl tert-butyl ether are used as solvent. The internal temperature is 74°C. The conversion based on **1** is 66.1%, the yield (**4 + 5 + 6** based on 1) is 21.0% and the selectivity of **4 + 5 + 6** is 31.7%.

### Comparison example 5: Preparation of a mixture of (E/Z)-3,7-dimethyl-2,6-octadiene nitrile, (E/Z)-3,7-dimethyl-3,6-octadiene nitrile and 7-methyl-3-methylene-6-octene nitrile in diisopropyl ether

In an analogous manner to examples 1-3 example 5 is carried out, whereby 10 ml of diisopropyl ether are used as solvent. The internal temperature is 83°C. The conversion based on **1** is 87.1%, the yield (**4 + 5 + 6** based on 1) is 39.3% and the selectivity of **4 + 5 + 6** is 45.1%.

### Condensation examples 6-10: Variation of the co-base 1

### Example 6: Preparation of a mixture of (E/Z)-3,7-dimethyl-2,6-octadiene nitrile, (E/Z)-3,7-dimethyl-3,6-octadiene nitrile and 7-methyl-3-methylene-6-octene nitrile in n-heptane using ammonium acetate as co-base 1

6.4 g (50 mmol) of 6-methyl-5-hepten-2-one (MH, 1), 4.5 g (52.5 mmol) of cyanoacetic acid (**2**), 5 ml (62.5 mmol) of pyridine, 10 ml of n-heptane and 393 mg (5 mmol) of ammonium acetate are filled into a 50 ml glass reactor (double jacketed) with reflux condenser, dean stark water separator and stirrer. The mixture is stirred at 600 rpm and heated to reflux temperature (112 - 116°C). The mixture is held under reflux and an Argon flow for 8 hours including water and carbon dioxide removal. After cooling (to 21°C) the reaction mixture is concentrated under reduced pressure (20 mbar, 40°C) and the brown residue is diluted in 20 ml of n-heptane. 10 ml of ice-cooled 6% w/w sulfuric acid is added dropwise over a period of 10 minutes. The mixture is stirred for 30 minutes at 20°C, followed by extraction with n-heptane. The n-heptane layer is washed with an aqueous 1 M sodium hydrogencarbonate solution (3 x 10ml) and further washed with water (3 x 10 ml). The organic solution is dried over 4 g of sodium sulfate, followed by removal of the solvent (20 mbar, 50°C) to give 7.4 g of a mixture of isomers **4, 5** and **6** (purity 88.8% analysed by GC, yield based on **1:** 87.7%, selectivity of **4 + 5 + 6:** 89.9%).

### Composition of the crude product as analysed by GC:

(*Z*)-3,7-dimethylocta-2,6-dienenitrile (**4b**) + 7-methyl-3-methyleneoct-6-enenitrile (**6**): 13.0% GC
(*Z*)-3,7-dimethylocta-3,6-dienenitrile (**5b**): 6.6% GC
(*E*)-3,7-dimethylocta-2,6-dienenitrile (**4a**) 35.0% GC
(*E*)-3,7-dimethylocta-3,6-dienenitrile (**5a**): 34.2% GC

### Example 7: Preparation of a mixture of (E/Z)-3,7-dimethyl-2,6-octadiene nitrile, (E/Z)-3,7-dimethyl-3,6-octadiene nitrile and 7-methyl-3-methylene-6-octene nitrile in n-heptane using ammonium acetate as co-base 1

Example 6 is repeated, but only 0.5 mmol (0.01 eq based on the molar amount of 1) of ammonium acetate are used. The results are as follows: conversion based on **1** is 92.2%, the yield **(4 + 5 + 6** based on **1)** is 80.1% and the selectivity of **4 + 5 + 6** is 86.8%.

### Comparison examples 8-10

### Comparison example 8: Preparation of a mixture of (E/Z)-3,7-dimethyl-2,6-octadiene nitrile, (E/Z)-3,7-dimethyl-3,6-octadiene nitrile and 7-methyl-3-methylene-6-octene nitrile in n-heptane using 4-(dimethylamino)pyridine as co-base 1

Example 6 is repeated, but 5 mmol (0.1 eq based on the molar amount of **1**) of 4-(dimethylamino)pyridine are used as co-base 1. The results are as follows: conversion based on **1** is 20.0%, the yield (**4 + 5 + 6** based on **1**) is 11.2% and the selectivity of **4 + 5 + 6** is 56.0%.

### Comparison example 9: Preparation of a mixture of (E/Z)-3,7-dimethyl-2,6-octadiene nitrile, (E/Z)-3,7-dimethyl-3,6-octadiene nitrile and 7-methyl-3-methylene-6-octene nitrile in n-heptane using 1,4-diaza-bicyclo[2.2.2]octane as co-base 1

Example 6 is repeated, but 5 mmol (0.1 eq based on the molar amount of 1) of 1,4-diaza-bicyclo[2.2.2]octane are used as co-base 1. The results are as follows: conversion based on 1 is 19.8%, the yield (**4 + 5 + 6** based on **1**) is 10.2% and the selectivity of **4 + 5 + 6** is 51.5%.

### Comparison example 10: Preparation of a mixture of (E/Z)-3,7-dimethyl-2,6-octadiene nitrile, (E/Z)-3,7-dimethyl-3,6-octadiene nitrile and 7-methyl-3-methylene-6-octene nitrile in n-heptane using 1,8-diaza-bicyclo-[5.4.0]undecene-7 as co-base 1

Example 6 is repeated, but 5 mmol (0.1 eq based on the molar amount of **1**) of 1,8-diaza-bicyclo-[5.4.0]undecene-7 are used as co-base 1. The results are as follows: conversion based on **1** is 25.1%, the yield (**4 + 5 + 6** based on **1**) is 16.4% and the selectivity of **4 + 5 + 6** is 65.3%.

### Condensation examples 11-16: Variation of the amount of base

The results are summarized in **table 1** below. The examples are performed as described in Example 2 whereby the amount of pyridine is used as given in **table 1.**

**Table 1**

| **Example** | **Amount of pyridine [eq based on amount of 1]** | **Conversion [%] based on 1** | **Yield of 4 + 5 + 6 [%]** | **Selectivity of 4 + 5 + 6 [%]** |
|---|---|---|---|---|
| **11** | 0 | 92.0 | 31.6 | 34.4 |
| **12** | 0.25 | 93.5 | 42.5 | 45.5 |
| **13** | 0.50 | 98.1 | 83.6 | 85.3 |
| **14** | 0.75 | 96.6 | 85.4 | 88.4 |
| **15** | 1.0 | 97.9 | 83.7 | 85.5 |
| **2** | 1.25 | 98.6 | 86.2 | 87.4 |
| **16** | 1.5 | 98.8 | 86.3 | 87.4 |

It was found that increasing the amount of pyridine above 1.25 eq did not improve the yield (example 16). An amount of pyridine between 0.5 and 1.25 eq showed to be optimal.

### Condensation examples 17-20: Variation of the amount of co-base 1

The results are summarized in **table 2** below. The examples are performed as described in Example 2 whereby the amount of the co-base 1-4-diaminobutane is used as given in **table 2,** and whereby the experiments are performed under a nitrogen flow.

**Table 2**

| **Example** | **Amount of 1,4-diaminobutane [eq based on amount of 1]** | **Conversion [%] based on 1** | **Yield of 4 + 5 + 6 [%]** | **Selectivity of 4 + 5 + 6 [%]** |
|---|---|---|---|---|
| **17** | 0 | 48.8 | 28.1 | 57.6 |
| **18** | 0.01 | 90.4 | 82.2 | 90.9 |
| **19** | 0.02 | 94.3 | 80.8 | 85.7 |
| **20** | 0.04 | 95.2 | 81.9 | 86.0 |

The yield decreased significantly when the reaction is carried in absence of the co-base 1,4-diaminobutane (DAB). The optimal amount of the co-base proved to be 0.01 eq of 1,4-diaminobutane. Under this reaction condition, the mixture of isomers **4 + 5 + 6** was formed in 82.2 % yield (example 18).

No advantages were observed when the amount of DAB exceeds 0.01 equivalents.

### Condensation examples 21-27: Variation of the temperature when using toluene as solvent

In further experiments the influence of the reaction temperature on yield and selectivity was studied.

The examples are performed as described in example 1 with the following common reaction conditions:
Scale: 50 mmol
Reaction time: 10 hours
Cyanoacetic acid: 1.0 eq
Base: pyridine, 1.25 eq
Co-base 1: 1,4-diamino-butane, 0.01 eq
Solvent: toluene
Conc. of **1** in the reaction mixture: 3.4 mMol/ml
   Continuous removal of water and carbon dioxide
   The reactions were carried out under Ar.

The results are summarized in the following **table 3.**

**Table 3:**

| **Example** | **Internal Temperature [°C]** | **Pressure [mbar]** | **Conversion [%] based on 1** | **Yield of 4 + 5 + 6 [%]** | **Selectivity of 4 + 5 + 6 [%]** |
|---|---|---|---|---|---|
| **21** | 50 | 130 | 20.1 | 8.5 | 42.3 |
| **22** | 60 | 170 | 35.2 | 19.2 | 54.5 |
| **23** | 70 | 195 | 66.1 | 32.5 | 49.2 |
| **24** | 80 | 405 | 79.2 | 40.3 | 50.9 |
| **25** | 90 | 530 | 92.7 | 61.2 | 66.0 |
| **26** | 100 | 805 | 90.1 | 76.8 | 85.2 |
| **27** | 112 | 1010 | 92.9 | 77.1 | 83.0 |

The best yield of the mixture of isomers **4 + 5 + 6** was obtained in the temperature range 100 - 112°C (examples 26 and 27). Lowering of the reaction temperature did not improve the selectivity (examples 21-25).

### Condensation examples 28-35: Variation of the reaction time when using toluene or n-heptane as solvent

The reaction time was varied over 2 hours and 10 hours. A reaction time of 8 hours seems to be optimal (example 32 (toluene) and example 2 (n-heptane)). Under these conditions toluene as solvent was less suited (82.2% yield) compared to n-heptane (86.2% yield). The results are summarized in **table 4.**

The common reaction conditions were as follows:
Scale: 50 mmol
Cyanoacetic acid: 1.0 eq
Base: pyridine, 1.25 eq
Co-base1: 1,4-diamino-butane, 0.01 eq
Solvent: examples 28-33: toluene, examples 34-35 and 2: n-heptane
Internal temperature: 112°C in examples 28-33; 108°C in examples 34-35 and 2 Conc. of **1** in the reaction mixture: 3.4 mmol/ml
   Continuous removal of water and carbon dioxide
   The reactions were carried out under nitrogen.

**Table 4:**

| **Example** | **Reaction time [hours]** | **Conversion [%] based on 1** | **Yield of 4 + 5 + 6 [%]** | **Selectivity of 4 + 5 + 6 [%]** |
|---|---|---|---|---|
| **28** | 1 | 85.2 | 52.8 | 62.0 |
| **29** | 2 | 88.5 | 67.8 | 76.7 |
| **30** | 4 | 86.5 | 75.2 | 86.9 |
| **31** | 6 | 94.3 | 80.8 | 85.7 |
| **32** | 8 | 90.4 | 82.2 | 90.9 |
| **33** | 10 | 92.5 | 76.5 | 82.7 |
| **34** | 4 | 93.1 | 80.4 | 86.3 |
| **35** | 6 | 98.7 | 85.0 | 86.1 |
| **2** | 8 | 98.6 | 86.2 | 87.4 |

### Condensation examples 36-37: Solvent-free condensation

6.4 g (50 mmol) of 6-methyl-5-hepten-2-one (MH, **1**), 4.5 g (52.5 mmol) of cyanoacetic acid (**2**), 5 ml (62.5 mmol) of pyridine and 51 µl (0.5 mmol) of 1,4-diaminobutane are filled into a 50 ml glass reactor (double jacketed) with reflux condenser, dean stark water separator and stirrer. The reaction was held at 112-116°C for the hours given in **table 5** under Ar as described in example 1.

**Table 5:**

| **Example** | **Reaction time [hours]** | **Conversion [%] based on 1** | **Yield of 4 + 5 + 6 [%]** | **Selectivity of 4 + 5 + 6 [%]** |
|---|---|---|---|---|
| **36** | 6 | 77.6 | 74.1 | 95.5 |
| **37** | 9 | 79.4 | 76.4 | 96.2 |

Under these conditions the isomers **4 + 5 + 6** were synthesized in 74 - 76% yield based on **1** (selectivity 96%). A selectivity of 96% was achieved at a partial conversion of **1** (78 - 79%).

### Condensation examples 38-44: Variation of the amount of cyanoacetic acid

The results are summarized in **table 6** below. The examples are performed as described in Example 2 whereby the amount of cyanoacetic acid is used as given in **table 6.**

**Table 6**

| **Example** | **Amount of cyanoacetic acid [moleq based on 1]** | **Conversion [%] based on 1** | **Yield of 4 + 5 + 6 [%]** | **Selectivity of 4 + 5 + 6 [%]** |
|---|---|---|---|---|
| **2** | 1.00 | 98.6 | 86.2 | 87.4 |
| **38** | 1.05 | 99.1 | 94.1 | 94.9 |
| **39** | 1.05 | 99.3 | 93.0 | 93.7 |
| **40** | 1.05 | 99.9 | 88.2 | 88.3 |
| **41** | 1.05 | 99.4 | 91.2 | 91.8 |
| **42** | 1.10 | 99.7 | 95.6 | 95.9 |
| **43** | 1.10 | 99.9 | 91.3 | 91.4 |
| **44** | 1.15 | 99.7 | 85.5 | 85.8 |

The amount of cyanoacetic acid (**2**) was varied between 1.0 and 1.15 moleq, based on **1.** It was found that a small excess of **2** (1.05 equivalents) increases the yield on the mixture of the isomers **4** + **5** + **6:** 94.1% yield at 99.1% conversion (example 38). Compared to example 2 the yield was increased by 8%.

This experiment was repeated several times (examples 39-41) and the results showed variation in yields (88.2% to 94.1%). No advantages were observed when the amount of **2** exceeds 1.05 equivalents.

### Condensation examples 45-48: Variation of the dosage time of 6-methyl-5-hepten-2-one (MH)

The dosage time of the starting material 1 was also tested at reflux temperature in n-heptane (108°C) in the range of 2 to 8 hours. The examples 45-48 are carried out in the same manner as example 2, but in example 48 the reaction time was 10 hours instead of 8 hours. The results are summarized in **table 7** below. A significant influence of the dosage time was not observed.

**Table 7**

| **Example** | **Dosage time [hours]** | **Conversion [%] based on 1** | **Yield of 4 + 5 + 6 [%]** | **Selectivity of 4 + 5 + 6 [%]** |
|---|---|---|---|---|
| **45** | 2 | 94.5 | 82.7 | 87.5 |
| **46** | 4 | 93.1 | 80.4 | 86.4 |
| **47** | 6 | 96.9 | 83.5 | 86.2 |
| **48** | 8 | 97.1 | 83.2 | 85.7 |

### Condensation examples 49-51: Variation of the dosage time of cyanoacetic acid

The dosage time of **2** was also tested at reflux temperature in n-heptane (108°C) in the range of 3 to 8 hours. Therefore **2** was melted at 70°C and the dosage time was varied from 3 hours to 8 hours. The examples 49-51 are carried out in the same manner as example 2, but in example 51 the reaction time was 10 hours instead of 8 hours. The results are summarized in **table 8** below. A prolonged dosage time of **2** into the reaction mixture improves slightly the yield. The mixture of the isomers **4 + 5 + 6** was formed in 85 - 86% yield.

**Table 8**

| **Example** | **Dosage time [hours]** | **Conversion [%] based on 1** | **Yield of 4 + 5 + 6 [%]** | **Selectivity of 4 + 5 + 6 [%]** |
|---|---|---|---|---|
| **49*** | 3 | 98.0 | 85.2 | 86.9 |
| **50** | 6 | 97.9 | 84.9 | 86.7 |
| **51** | 8 | 98.5 | 86.2 | 87.5 |

| | | | | |
|---|---|---|---|---|
| * Composition of the product P as analyzed by gas chromatography (GC): | | | | |

(Z)-3,7-dimethylocta-2,6-dienenitrile (**4b**) + 7-methyl-3-methyleneoct-6-enenitrile (**6**): 16.1 % GC
(*Z*)-3,7-dimethylocta-3,6-dienenitrile (**5b**): 5.9 % GC
(*E*)-3,7-dimethylocta-2,6-dienenitrile (**4a**): 24.1 % GC
(*E*)-3,7-dimethylocta-3,6-dienenitrile (**5a**): 34.2 % GC

### Condensation examples 52-56: Effect of the water and carbon dioxide removal

Investigations to improve the removal of water and carbon dioxide were performed by a weak flow of Ar or N₂. The water formed during the reaction was trapped in a Dean-Stark apparatus and the carbon dioxide was removed with the inert gas flow. The reactions were carried out in n-heptane at reflux temperature (108 °C) using an amount of 1.05 eq of cyanoacetic acid (**2**) in an analogous manner to example 2. The flow of argon was varied and monitored by a gas bubbler or a flow controller. The results are summarized in **table 9.**

**Table 9**

| **Example** | **Ar flow flow controller** | **flow** | **Results Conversion of 1** | **Yield of 4 + 5 + 6** | **Selectivity of 4 + 5 + 6** |
|---|---|---|---|---|---|
| | | **[L/hour]** | | | |
| **52** | | none | 96.7 | 57.3 | 59.0 |
| **53** | gas bubbler | 4 to 10 | 89.9 | 74.6 | 83.0 |
| **54** | gas bubbler | 15 to 30 | 99.1 | 94.1 | 94.9 |
| **55** | flow controller | 20 | 98.8 | 92.9 | 93.9 |
| **56** | flow controller | 40 | 98.8 | 90.8 | 91.9 |

It was found that traces of water and carbon dioxide affected dramatically the yield of the reaction. The experiment carried out without water and carbon dioxide removals results in the lowest yield (example 52, yield 57.3 %). The highest yields were obtained when a strong Ar flow (20 to 40 L/hour) was applied. Under these conditions a nearly quantitative conversion of 1 to a mixture of isomers **4** + **5** + **6** in 90 - 94% yield were obtained (example 54).

### Detailed description of example 54

In a 50-ml double-jacketed glass reactor equipped with a Dean Stark water separator and a reflux condenser are placed 6.4 g (50 mmol) of 6-methyl-5-hepten-2-one (**1**), 4.5 g (52.5 mmol) of cyanoacetic acid (**2**), 5 ml (62.5 mmol) of pyridine, 10 ml of n-heptane and 51 µl (0.5 mmol) of 1,4-diamino-butane (**3**). The mixture is stirred at 600 rpm and heated to reflux temperature (112°C). The mixture is held under reflux and a strong Argon flow (15 to 30 L/hour through a gas dispenser tube) for 8 hours including water and carbon dioxide removal. After cooling (to 20°C), the reaction mixture was evaporated under reduced pressure (20 mbar, 50°C). 8.4 g of a brown liquid was obtained (content: 84.1 % of geranyl nitrile isomers, analyzed by GC (ISTD).

The isolated yield was 94.1 % based on **1** and the selectivity was 94.9%.

### Composition of the product:

(Z)-3,7-dimethylocta-2,6-dienenitrile (**4b**) + 7-methyl-3-methyleneoct-6-enenitrile (6): 17.3 % GC
(*Z*)-3,7-dimethylocta-3,6-dienenitrile (**5b**): 6.8 % GC
(*E*)-3,7-dimethylocta-2,6-dienenitrile (**4a**): 24.7 % GC
(*E*)-3,7-dimethylocta-3,6-dienenitrile (**5a**): 35.3 % GC

### Condensation examples 57-58: Scale-up examples

The synthesis of a mixture of isomers **4** + **5** + **6** was performed on preparative scale (250 mmol of 1). The optimized conditions (solvent: n-heptane, reaction temperature: 110 - 116°C, reaction time: 8 hours, 312.5 mmol of pyridine, 2.5 mmol of DAB, 1.05 to 1.10 eq of **2,** argon flow 20 L/h) were applied. The crude product was purified by distillation. The purified products were analyzed by GC and selected samples by ¹H NMR. The product (mixture of **4** + **5** + **6**) was isolated in 84 - 85% yield and 96 - 98% purity. The results are summarized in **tables 10 and 11.**

**Table 10**

| **Example** | | **57** | **58** |
|---|---|---|---|
| | | | |
| methyl-heptenone (**1**) | | | |
| Quality | [% GC] | 99.0 | 99.0 |
| Scale | [mmol] | 250 | 250 |
| | | | |
| Reactions parameters | | | |
| Solvent | - | n-heptane | n-heptane |
| Base | | pyridine | pyridine |
| | [eq.] | 1.25 | 1.25 |
| Co-base | | DAB | DAB |
| | [eq.] | 0.01 | 0.01 |
| Cyanoacetic acid (**2**) | [eq.] | 1.05 | 1.1 |
| Concentration of **1** | [Mol/ml] | 3.4 | 3.4 |
| Reaction temperature | [°C] | 115 | 115 |
| Reaction time | [hours] | 8 | 8 |
| Argon flow | [L/h] | 20 | 30 |
| | | | |
| Results | | | |
| Conversion of **1** | [%] | 97.8 | 98.5 |
| Yield of **4** + **5** + **6** | [%] | 91.2 | 92.3 |
| Selectivity of **4** + **5** + **6** | [%] | 93.3 | 93.7 |
| Yield of **4** + **5** + **6** | [%] | 84.9 | 83.5 |
| | | | |
| GC Analysis | | | |
| methyl-heptenone (**1**) | [% GC] | 1.3 | 0.1 |
| E-3,7-dimethylocta-2,6-dienenitrile (**4a**) | [% GC] | 7.0 | 7.2 |
| Z-3,7-dimethylocta-2,6-dienenitrile (**4b**) + 7-methyl-3-methyleneoct-6-enenitrile (**6**) | [% GC] | 34.4 | 26.7 |
| E-3,7-dimethylocta-3,6-dienenitrile (**5a**) | [% GC] | 37.9 | 48.2 |
| Z-3,7-dimethylocta-3,6-dienenitrile (**5b**) | [% GC] | 16.8 | 16.0 |
| ∑ geranyl nitrile isomers | [% GC] | 96.1 | 98.1 |

**Table 11**

| **Example** | | **58** |
|---|---|---|
| | | |
| ¹H NMR Analysis | | |
| (E/Z)-3,7-dimethylocta-2,6-dienenitrile (**4**) | [%] | 12.0 |
| 7-methyl-3-methyleneoct-6-enenitrile (**6**) | [%] | 26.0 |
| (E/Z)-3,7-dimethylocta-3,6-dienenitrile (**5**) | [%] | 67.0 |
| ∑ geranyl nitrile isomers | [%] | 105.0 |

### Detailed description of the scaled-up process

In a 200 ml double-jacketed glass reactor equipped with a Dean Stark water separator, a reflux condenser and a stirrer 31.9 g (250 mmol) of methyl-heptenone (**1**), 22.6 g (262.5 mmol) of cyanoacetic acid (**2**), 25.2 ml (312.5 mMol) of pyridine, 50 ml of n-heptane and 256 µl (2.5 mMol) of 1,4-diamino-butane (**3**) are placed. The mixture is stirred at 500 rpm and heated to reflux temperature (115°C). The mixture is held under reflux and an Argon flow (20 L/h through a gas dispenser tube) for 8 hours including water and carbon dioxide removal (the double jacketed water separator is cooled to 10°C) The reaction mixture was cooled to 40°C and the solvent (n-heptane) and the base (pyridine) are distilled under reduced pressure (20 mbar, 40°C). The brown liquid residue is diluted in 100 ml of n-heptane and the mixture cooled down to 10°C. 25 ml of ice-cooled 6% w/w sulfuric acid are added drop-wise over a period of 30 minutes. The mixture is stirred for 30 minutes at 10°C, followed by extraction with n-heptane. The n-heptane layer is washed with an aqueous 1 M sodium hydrogencarbonate solution (3 x 25ml) and further washed with water (3 x 25 ml). The organic solution is dried over 20 g of sodium sulphate, followed by removal of the solvent (20 mbar, 50°C). Afterwards the crude product is distilled. A mixture of geranonitrile isomers boiling at 70°C / 0.07 mbar is collected. The colourless liquid product is analysed by GC ISTD.

Comparison of the crude product analysis **(table 10**) shows that an isomerization of **4** to **5** and **6** occur during the distillation. The content of E/Z-3,7-dimethylocta-2,6-dienenitrile (**4**) decreases by 40% and the content of 7-methyl-3-methyleneoct-6-enenitrile (**6**) and (E/Z)-3,7-dimethylocta-3,6-dienenitrile (**5**) increases by 24 and 22%, respectively.

### II. Isomerization examples

Two charges of geranyl nitrile isomers were heated at 120°C during 20 hours in the presence of 1,4-diamino-butan (0.01 Mol%). The *E*/*Z*-isomerization was measured by ¹H-NMR spectroscopy. The results are summarized in **tables 12 and 13.**

The isomer **6** (7-methyl-3-methylene-oct-6-enenitrile) was almost completely converted to **4** and the isomer **5** (3,7-dimethylocta-3,6-dienenitrile) was partially converted to **4.**

**Table 12**

| **Example** | **Time [hours]** | **4 [% ¹H NMR]** | **5 [% ¹H NMR]** | **6 [% ¹H NMR]** |
|---|---|---|---|---|
| Isomerization of geranyl nitrile isomers without co-base | | | | |
| 1 | 0 | 12.3 | 54.8 | 32.9 |
| 2 | 20 | 12.9 | 56.0 | 31.0 |

| Isomerization of geranyl nitrile isomers in presence of DAB (**4**) | | | | |
|---|---|---|---|---|
| 3 | 0 | 12.3 | 54.8 | 32.9 |
| 4 | 1 | 49.1 | 48.3 | 2.6 |
| 5 | 2 | 58.4 | 40.0 | 1.6 |
| 6 | 8 | 66.2 | 32.0 | 1.8 |
| 7 | 20 | 80.3 | 18.3 | 1.4 |

**Table 13**

| **Example** | **Time [hours]** | **4 [% ¹H NMR]** | **5 [% ¹H NMR]** | **6 [% ¹H NMR]** |
|---|---|---|---|---|
| Isomerization of geranyl nitrile isomers without co-base | | | | |
| 1 | 0 | 53.1 | 45.9 | 1.0 |
| 2 | 20 | 68.2 | 30.1 | 1.6 |

| Isomerization of geranyl nitrile isomers in presence of DAB (**4**) | | | | |
|---|---|---|---|---|
| 3 | 0 | 53.1 | 45.9 | 1.0 |
| 4 | 1 | 58.3 | 39.5 | 2.1 |
| 5 | 2 | 63.3 | 34.8 | 2.0 |
| 6 | 8 | 69.2 | 29.1 | 1.7 |
| 7 | 20 | 88.5 | 10.9 | 0.6 |

The isomer **5** (3,7-dimethylocta-3,6-dienenitrile) is almost completely converted to an E/Z-mixture of **4.** The result also indicates that the co-base promotes markedly the isomerization of the 7-methyl-3-methyleneoct-6-enenitrile and 3,7-dimethylocta-3,6-dienenitrile to geranyl nitrile/neryl nitrile (**4**).

This also proves that the isomerization can be controlled by addition of a catalyst such as 1,4-diamino-butan (**3**).

### Hydrogenation examples

3,7-dimethyl-2,6-octadiene nitrile is purchased from Sigma Aldrich; purity 97%, as a mix of 2E- and 2Z-isomers with a ratio of approximately 1 : 1.

### Examples 1-5: Hydrogenation of (E,Z)-3,7-dimethyl-2,6-octadiene nitrile

### Small scale experiments

### Example 1

The mixture of geranyl nitrile and neryl nitrile, the solvent, catalyst and additive (if required) are added in the amounts given in **table 14** to a glass reactor. The reactor is sealed and is purged with argon 5 times (by pressurising to 5 bar followed by release of the pressure) and 3 times with hydrogen (pressurise to 3 bar then release). The reaction mixture is heated to the desired temperature, pressurised to the desired hydrogen pressure and stirred at 1000 rpm. At the end of the reaction the pressure is released and the reactor purged 3 times with argon. After filtration to remove the catalyst, the reaction mixture is analysed by GC to determine conversion and selectivity.

### Comparison examples A-D

These experiments are performed in the same manner as example 1 with the solvent, the catalyst, the additive and the reaction conditions as given in **table 14.**

### Larger scale experiments

### Example 2

This experiment is carried out in an analogous manner as example 1. Details about the solvent, the catalyst, the additive and the reaction conditions are given in **table 15.**

### Examples 3-7

The mixture of geranyl nitrile and neryl nitrile, the solvent, catalyst and additive are added in the amounts given in **tables 15 to 17** to a 500 ml stainless steel reactor. The reactor is sealed and is purged with nitrogen 3 times (by pressurising to 5 bar followed by release of the pressure) and 3 times with hydrogen (pressurise to 5 bar then release). The reaction mixture is heated to the desired temperature, pressurised to the desired hydrogen pressure and stirred at 1000 rpm. At the end of the reaction the pressure is released and the reactor purged once with nitrogen. After filtration to remove the catalyst, the reaction mixture is analysed by GC to determine conversion and selectivity.

The results (conversion and selectivity) of the experiments 1-7 (according to the invention) and the comparison examples A-D are listed in **tables 14, 15, 16 and 17.**

### Example 8 - Catalyst re-use experiments

The first reaction was performed as described in examples 3-7 with 20 g of unsaturated nitrile, 200 g 2-propanol and 9 g of nickel catalyst. At the end of the reaction the pressure was released and the reactor was purged once with nitrogen. The catalyst remained in the reactor and the product solution was removed from the reactor via a dip-tube fitted with a metal frit. The catalyst was washed by 150-200 g 2-propanol. A new solution of unsaturated nitrile in 2-propanol was added through the dip-tube. The reactor was purged with nitrogen 3 times (by pressurising to 5 bar followed by release of the pressure) and 3 times with hydrogen (pressurise to 5 bar then release). The reaction mixture was heated to the desired temperature, pressurised to the desired hydrogen pressure and stirred at 1000 rpm.

The results of example 8 can be found in table 18.

**Table 14: Hydrogenation of a 1:1 mixture of 3,7-dimethyl-2(Z),6-octadiene nitrile and 3,7-dimethyl-2(E),6-octadiene nitrile**

| Example 1: according to the invention; examples A-D: comparison examples; T = Temperature | | | | | | | | | |
|---|---|---|---|---|---|---|---|---|---|
| **Example** | **Amount of Starting Material** | **Solvent (amount)** | **Catalyst (amount)** | **Additive (amount)** | **Time (hours)** | **T (°C)** | **Pressure H₂ (bar absolute)** | **Conversion (%)** | **Selectivity (%)** |
| **1** | 200 mg | Heptane (2.0 g) | Nickel Alloy Johnson Matthey A4000 (160 mg) | 2,2'-(ethylenedithio)-diethanol (6 mg) | 25 | 50 | 6 | 98 | 91 |
| **A** | 270 mg | Heptane (2.7 g) | 5% Pd/C Evonik E 101 N/D (5 mg) | Triethylamine (69 mg) | 6 | 50 | 6 | 20 | 19 |
| **B** | 360 mg | Heptane (3.4 g) | 5% Pd/C Evonik E 101 N/D (5 mg) | DBU (2 mg) | 26 | 80 | 11 | 3 | 2 |
| **C** | 200 mg | Dichloromethane (2.2 g) | 5% Pd/CaCO₃ Evonik | None | 22 | 25 | 6 | 22 | 8 |
| **D** | 1.0 g | Heptane (10 g) | 5% Pd/Al₂O₃ Evonik | None | 24 | 25 | 11 | 100 | 35 |

**Table 15: Hydrogenation of a 1:1 mixture of 3,7-dimethyl-2(Z),6-octadiene nitrile and 3,7-dimethyl-2(E),6-octadiene nitrile**

| Examples 2-5: according to the invention, T = temperature | | | | | | | | | |
|---|---|---|---|---|---|---|---|---|---|
| **Example** | **Starting Material** | **Solvent (amount)** | **Catalyst (amount)** | **Additive (amount)** | **Time (hours)** | **T (°C)** | **Pressure H₂ (bar absolute)** | **Conversion (%)** | **Selectivity (%)** |
| **2** | 2.0 g | Heptane (20 g) | Nickel Alloy Johnson Matthey A4000 (1.0 g) | 2,2'-(ethylenedithio)-diethanol (50 mg) | 65 | 50 | 6 | 100 | 91 |
| **3** | 20.0 g | Heptane (200 g) | Nickel Alloy Johnson Matthey A4000 (10 g) | 2,2'-(ethylenedithio)-diethanol (450 mg) | 49 | 50 | 6 | 100 | 95 |
| **4** | 20.0 g | Heptane (200 g) | Nickel Alloy Johnson Matthey A4000 (9 g) | 2,2'-(ethylenedithio)-diethanol (360 mg) | 40 | 50 | 6 | 94 | 87 |
| **5** | 10.0 g | Heptane (200 g) | Nickel Alloy Johnson Matthey A4000 (6 g) | 2,2'-(ethylenedithio)-diethanol (180 mg) | 21 | 50 | 6 | 97 | 88 |

**Table 16: Hydrogenation of a 1:1 mixture of 3,7-dimethyl-2(Z),6-octadiene nitrile and 3,7-dimethyl-2(E),6-octadiene nitrile**

| Examples 6: according to the invention, T = temperature | | | | | | | | | |
|---|---|---|---|---|---|---|---|---|---|
| **Example** | **Starting Material** | **Solvent (amount)** | **Catalyst (amount)** | **Additive (amount)** | **Time (hours)** | **T (°C)** | **Pressure H₂ (bar absolute)** | **Conversion (%)** | **Selectivity (%)** |
| **6a** | 20.0 g | Toluene (20 g) | Nickel Alloy Johnson Matthey A4000 (9 g) | 2,2'-(ethylenedithio)-diethanol (360 mg) | 25 | 50 | 6 | 96 | 88 |
| **6b** | 20.0 g | Ethyl acetate (200 g) | Nickel Alloy Johnson Matthey A4000 (9 g) | 2,2'-(ethylenedithio)-diethanol (360 mg) | 42 | 50 | 6 | 99 | 90 |
| **6c** | 20.0 g | 2-propanol (200 g) | Nickel Alloy Johnson Matthey A4000 (9 g) | 2,2'-(ethylenedithio)-diethanol (360 mg) | 20 | 50 | 6 | 99 | 92 |
| **6d** | 20.0 g | Tetrahydrofuran (200 g) | Nickel Alloy Johnson Matthey A4000 (9 g) | 2,2'-(ethylenedithio)-diethanol (180 mg) | 7 | 50 | 6 | 99 | 86 |

**Table 17: Hydrogenation of a 1:1 mixture of 3,7-dimethyl-2(Z),6-octadiene nitrile and 3,7-dimethyl-2(E),6-octadiene nitrile**

| Examples 7: according to the invention, T = temperature | | | | | | | | | |
|---|---|---|---|---|---|---|---|---|---|
| **Example** | **Starting Material** | **Solvent (amount)** | **Catalyst (amount)** | **Additive (amount)** | **Time (hours)** | **T (°C)** | **Pressure H₂ (bar absolute)** | **Conversion (%)** | **Selectivity (%)** |
| **7a** | 20.0 g | Heptane (200 g) | Nickel Alloy - Johnson Matthey A4000 (9 g) | Diethyl disulfide (240 mg) | 5.5 | 50 | 6 | 82 | 67 |
| **7b** | 20.0 g | Heptane (200 g) | Nickel Alloy - Johnson Matthey A4000 (9 g) | Diethylsulfide (180 mg) | 2 | 50 | 6 | 79 | 56 |
| **7c** | 20.0 g | Heptane (200 g) | Nickel Alloy - Johnson Matthey A4000 (9 g) | 1,8-Diazabicyclo[5.4.0]-undec-7-ene (DBU, 1.5 g) | 6 | 50 | 6 | 92 | 63 |
| **7d** | 20.0 g | Heptane (200 g) | Nickel Alloy - Johnson Matthey A4000 (9 g) | Diphenyl sulfide (730 mg) | 2 | 50 | 6 | 87 | 59 |
| **7e** | 20.0 g | Heptane (200 g) | Nickel Alloy - Johnson Matthey A4000 (9 g) | 1,4-diaminobutane (350 mg) | 4 | 50 | 6 | 85 | 67 |
| **7f** | 20.0 g | Heptane (200 g) | Nickel Alloy - Johnson Matthey A4000 (9 g) | Ethylenediamine (240 mg) | 14 | 50 | 6 | 90 | 78 |
| **7g** | 20.0 g | Heptane (200 g) | Nickel Alloy - Johnson Matthey A4000 (9 g) | Thiophene (670 mg) | 3.4 | 50 | 6 | 97 | 84 |

**Table 18: Hydrogenation of a 1:1 mixture of 3,7-dimethyl-2(Z),6-octadiene nitrile and 3,7-dimethyl-2(E),6-octadiene nitrile**

| Examples 8: according to the invention, T = temperature | | | | | | | | | |
|---|---|---|---|---|---|---|---|---|---|
| **Example** | **Starting Material** | **Solvent (amount)** | **Catalyst added (amount)** | **Additive added (amount)** | **Time (hours)** | **T (°C)** | **Pressure H₂ (bar absolute)** | **Conversion (%)** | **Selectivity (%)** |
| **8a** | 20.0 g | 2-propanol (200g) | Nickel Alloy Johnson Matthey A4000 (9 g) | 2,2'-(ethylenedithio)-diethanol (360 mg) | 15 | 50 | 6 | 99 | 93 |
| **8b** | 20.0 g | 2-propanol (200g) | None (catalyst reused 1^{st} time) | none | 24 | 50 | 6 | 99 | 90 |
| **8c** | 20.0 g | 2-propanol (200g) | None (catalyst reused 2^{nd} time) | none | 24 | 50 | 6 | 99 | 91 |
| **8d** | 20.0 g | 2-propanol (200g) | None (catalyst reused 3^{rd} time) | none | 25 | 50 | 6 | 99 | 91 |

## Claims

1. A process for the manufacture of a compound of formula II 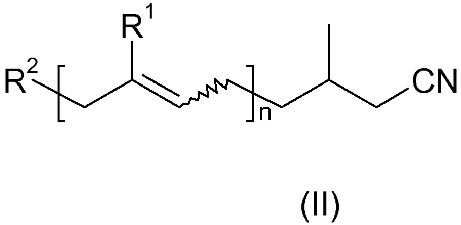 by reduction of a compound of formula I 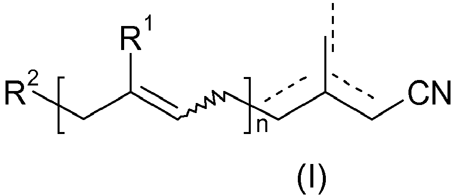 with hydrogen in the presence of a catalyst comprising nickel, wherein an additive is present, and
wherein n is either 1 or 2, preferably wherein n is 1;
wherein R¹ is linear C₁₋₄ alkyl or branched C₃₋₄ alkyl,
preferably wherein R¹ is methyl or ethyl, and
wherein R² is hydrogen or linear C₁₋₄ alkyl or branched C₃₋₄ alkyl,
preferably wherein R² is hydrogen or methyl, and
more preferably wherein either R¹ is methyl or R² is hydrogen
wherein the additive is selected from the group of additives containing a nitrogen or a sulfur atom,
wherein the additive containing a sulfur atom is selected from the group of aromatic disulfides, aliphatic disulfides, aromatic sulfides, heteroaromatic sulfides, aliphatic sulfides, aromatic thiols, heteroaromatic thiols, aliphatic thiols, each containing a total number of 2-15 carbon atoms and a total number of 1 or 2 sulfur atoms, respectively, and
wherein the additive containing a nitrogen atom is selected from the group of aliphatic primary amines containing a total number of 3-10 carbon atoms and 1-3 nitrogen atoms and C6-C10 bicyclic aliphatic amidines; and
wherein the reduction with hydrogen is carried out at an absolute hydrogen pressure in the range of 1 to 20 bar.

2. The process according to claim 1, wherein the reduction is carried out in an organic solvent.

3. The process according to claim 2, wherein the organic solvent is selected from the group consisting of aliphatic hydrocarbons, aromatic hydrocarbons, esters, ethers and alcohols, and any mixtures thereof, preferably wherein the organic solvent is selected from the group consisting of aliphatic hydrocarbons, alcohols and any mixtures thereof.

4. The process according to claim 2 and/or 3, wherein the organic solvent is n-heptane or 2-propanol or any mixture thereof, preferably wherein the organic solvent is 2-propanol.

5. The process according to any one or more of the preceding claims, wherein the additive is selected from the group of additives containing a sulfur atom.

6. The process according to claim 1, wherein the additive containing a nitrogen atom is selected from the group consisting of 1,8-diaza-bicyclo[5.4.0]undec-7-ene, ethylene diamine and 1,4-diaminobutane.

7. The process according to claim 1, wherein the additive containing a sulfur atom is selected from the group consisting of diethyl disulfide, diphenyl disulfide, diethyl sulfide, diphenyl sulfide, diethylendisulfide, thiophene and 2,2'-(ethylenedithio)-diethanol, preferably wherein the additive containing a sulfur atom is selected from the group consisting of diethyl disulfide, diethyl sulfide, diphenyl sulfide, thiophene and 2,2'-(ethylenedithio)diethanol, more preferably wherein the additive containing a sulfur atom is thiophene or 2,2'-(ethylenedithio)diethanol.

8. The process according to any one or more of the preceding claims, wherein the reduction with hydrogen is carried out at a temperature in the range of 10 to 100°C, preferably at a temperature in the range of 20 to 80°C, more preferably at a temperature in the range of 40 to 60°C, even more preferably at a temperature in the range of 45 to 55°C.

9. The process according to any one or more of the preceding claims, wherein the reduction with hydrogen is carried out at an absolute hydrogen pressure in the range of 2 to 11 bar, preferably at a hydrogen pressure in the range of 4 to 9 bar, more preferably at an absolute hydrogen pressure of 6 bar.

10. The process according to any one or more of the preceding claims, wherein the catalyst is of a nickel-alloy type, preferably wherein the catalyst comprises an amount of nickel in the range of 75 to 95% by weight and an amount of aluminium in the range of 0-15% by weight with the remainder made up by other metals such that the total weight sums to 100%.

11. The process according to claim 10, wherein the catalyst is promoted with iron or chromium or both, preferably in an amount of 0 to 10% by weight, based on the total weight of the catalyst.

12. The process according to any one or more of the preceding claims, wherein the amount of catalyst is in the range of 1 to 100 weight-%, preferably wherein the amount of catalyst is in the range of 10 to 80 weight-%, more preferably wherein the amount of catalyst is in the range of 30 to 60 weight-%, based on the amount of the compound of formula (I).

13. The process according to any one or more of the preceding claims, wherein the amount of the additive is in the range of 0.01 to 100 weight-%, preferably wherein the amount of the additive is in the range of 0.1 to 10 weight-%, more preferably wherein the amount of the additive is in the range of 0.2 to 2 weight-%, based on the amount of the catalyst.

14. The process according to any one or more of claims 2 to 13, wherein the amount of the solvent is chosen in such a way so that the solvent forms by weight 20 to 98% of the total solution to be hydrogenated, preferably wherein the amount of the solvent is chosen in such a way so that the solvent forms by weight 50 to 95% of the total solution to be hydrogenated, more preferably wherein the amount of the solvent is chosen in such a way so that the solvent forms by weight 70 to 90% of the total solution to be hydrogenated.

15. The process according to any one or more of the preceding claims, wherein pure H₂-gas or a gas mixture which comprises H₂ is used.

16. The process according to any one or more of the preceding claims, wherein the catalyst is re-used multiple times.

17. Use of a compound of formula (II) with the proviso that R² is not hydrogen if R¹ = methyl and n = 1 as aroma ingredient.

18. Use of a compound of formula (II) with the proviso that R² is not hydrogen if R¹ = methyl and n = 1 in flavour and/or fragrance applications.

19. A flavor or fragrance formulation comprising
(i) at least one compound of formula (II) with the proviso that R² is not hydrogen if R¹ = methyl and n = 1.

20. The flavor and fragrance formulation according to claim 19 comprising 0.0001 - 10 wt-% of at least one compound of formula (II), related to the total weight of the flavor and fragrance formulation.

21. The flavor and fragrance formulation according to claim 19 and/or 20, wherein the flavor and fragrance formulation is solid, gel-like or liquid.

22. The flavor and fragrance formulation according to any one or more of claims 19 - 21, wherein the flavor and fragrance formulation is a perfume, air care product, household product, laundry product, body care product or cosmetic product.

23. A method of improving, enhancing or modifying a flavor or fragrance formulation by means of addition thereto an olfactory acceptable amount of at least one compound of formula (II) with the proviso that R² is not hydrogen if R¹ = methyl and n = 1.

24. A compound of formula (II) 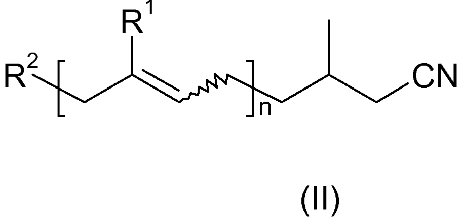 wherein n is either 1 or 2, preferably wherein n = 1; and
wherein R¹ is linear C₁₋₄ alkyl or branched C₃₋₄ alkyl,
preferably wherein R¹ is methyl or ethyl, and
wherein R² is hydrogen or linear C₁₋₄ alkyl or branched C₃₋₄ alkyl,
preferably wherein R² is hydrogen or methyl,
with the proviso that R² is not hydrogen if R¹ = methyl and n = 1 or 2.

## Patentansprüche

1. Verfahren zur Herstellung einer Verbindung der Formel II 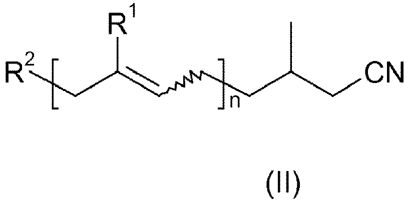 durch Reduktion einer Verbindung der Formel I 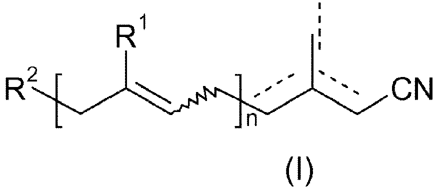 mit Wasserstoff in Gegenwart eines Nickel umfassenden Katalysators, wobei ein Additiv zugegen ist, und
wobei n entweder für 1 oder für 2 steht, vorzugsweise wobei n für 1 steht;
wobei R¹ für lineares C₁₋₄-Alkyl oder verzweigtes C₃₋₄-Alkyl steht,
vorzugsweise wobei R¹ für Methyl oder Ethyl steht, und
wobei R² für Wasserstoff oder lineares C₁₋₄-Alkyl oder verzweigtes C₃₋₄-Alkyl steht,
vorzugsweise wobei R² für Wasserstoff oder Methyl steht, und
weiter bevorzugt wobei entweder R¹ für Methyl steht oder R² für Wasserstoff steht;
wobei das Additiv aus der Gruppe von Additiven, die ein Stickstoff- oder ein Schwefelatom enthalten, ausgewählt wird,
wobei das Additiv, das ein Schwefelatom enthält, aus der Gruppe bestehend aus aromatischen Disulfiden, aliphatischen Disulfiden, aromatischen Sulfiden, heteroaromatischen Sulfiden, aliphatischen Sulfiden, aromatischen Thiolen, heteroaromatischen Thiolen und aliphatischen Thiolen, die jeweils eine Gesamtzahl von 2 bis 15 Kohlenstoffatomen und eine Gesamtzahl von 1 oder 2 Schwefelatomen enthalten, ausgewählt wird und
wobei das Additiv, das ein Stickstoffatom enthält, aus der Gruppe der aliphatischen primären Amine mit einer Gesamtzahl von 3-10 Kohlenstoffatomen und 1-3 Stickstoffatomen und bicyclischen aliphatischen C6-C10-Amidinen ausgewählt wird; und
wobei die Reduktion mit Wasserstoff bei einem absoluten Wasserstoffdruck im Bereich von 1 bis 20 bar durchgeführt wird.

2. Verfahren nach Anspruch 1, wobei die Reduktion in einem organischen Lösungsmittel durchgeführt wird.

3. Verfahren nach Anspruch 2, wobei das organische Lösungsmittel aus der Gruppe bestehend aus aliphatischen Kohlenwasserstoffen, aromatischen Kohlenwasserstoffen, Estern, Ethern und Alkoholen und Mischungen davon ausgewählt wird, vorzugsweise wobei das organische Lösungsmittel aus der Gruppe bestehend aus aliphatischen Kohlenwasserstoffen, Alkoholen und Mischungen davon ausgewählt wird.

4. Verfahren nach Anspruch 2 und/oder 3, wobei es sich bei dem organischen Lösungsmittel um n-Heptan oder 2-Propanol oder eine Mischung davon handelt, vorzugsweise wobei es sich bei dem organischen Lösungsmittel um 2-Propanol handelt.

5. Verfahren nach einem oder mehreren der vorhergehenden Ansprüche, wobei das Additiv aus der Gruppe der Additive, die ein Schwefelatom enthalten, ausgewählt wird.

6. Verfahren nach Anspruch 1, wobei das Additiv, das ein Stickstoffatom enthält, aus der Gruppe bestehend aus 1,8-Diazabicyclo[5.4.0]undec-7-en, Ethylendiamin und 1,4-Diaminobutan ausgewählt wird.

7. Verfahren nach Anspruch 1, wobei das Additiv, das ein Schwefelatom enthält, aus der Gruppe bestehend aus Diethyldisulfid, Diphenyldisulfid, Diethylsulfid, Diphenylsulfid, Diethylendisulfid, Thiophen und 2,2'-(Ethylendithio)diethanol ausgewählt wird, vorzugsweise wobei das Additiv, das ein Schwefelatom enthält, aus der Gruppe bestehend aus Diethyldisulfid, Diethylsulfid, Diphenylsulfid, Thiophen und 2,2'-(Ethylendithio)diethanol ausgewählt wird, weiter bevorzugt wobei es sich bei dem Additiv, das ein Schwefelatom enthält, um Thiophen oder 2,2'-(Ethylendithio)diethanol handelt.

8. Verfahren nach einem oder mehreren der vorhergehenden Ansprüche, wobei die Reduktion mit Wasserstoff bei einer Temperatur im Bereich von 10 bis 100 °C, vorzugsweise bei einer Temperatur im Bereich von 20 bis 80 °C, weiter bevorzugt bei einer Temperatur im Bereich von 40 bis 60 °C, noch weiter bevorzugt bei einer Temperatur im Bereich von 45 bis 55 °C, durchgeführt wird.

9. Verfahren nach einem oder mehreren der vorhergehenden Ansprüche, wobei die Reduktion mit Wasserstoff bei einem absoluten Wasserstoffdruck im Bereich von 2 bis 11 bar, vorzugsweise bei einem Wasserstoffdruck im Bereich von 4 bis 9 bar, weiter bevorzugt bei einem absoluten Wasserstoffdruck von 6 bar, durchgeführt wird.

10. Verfahren nach einem oder mehreren der vorhergehenden Ansprüche, wobei der Katalysator von einem Nickellegierungstyp ist, vorzugsweise wobei der Katalysator eine Nickelmenge im Bereich von 75 bis 95 Gew.-% und eine Aluminiummenge im Bereich von 0-15 Gew.-% umfasst, wobei der Rest aus anderen Metallen besteht, so dass sich das Gesamtgewicht zu 100 % summiert.

11. Verfahren nach Anspruch 10, wobei der Katalysator mit Eisen und/oder Chrom promotiert ist, vorzugsweise in einer Menge von 0 bis 10 Gew.-%, bezogen auf das Gesamtgewicht des Katalysators.

12. Verfahren nach einem oder mehreren der vorhergehenden Ansprüche, wobei die Katalysatormenge im Bereich von 1 bis 100 Gew.-% liegt, vorzugsweise wobei die Katalysatormenge im Bereich von 10 bis 80 Gew.-% liegt, weiter bevorzugt wobei die Katalysatormenge im Bereich von 30 bis 60 Gew.-% liegt, bezogen auf die Menge der Verbindung der Formel (I).

13. Verfahren nach einem oder mehreren der vorhergehenden Ansprüche, wobei die Menge des Additivs im Bereich von 0,01 bis 100 Gew.-% liegt, vorzugsweise wobei die Menge des Additivs im Bereich von 0,1 bis 10 Gew.-% liegt, weiter bevorzugt wobei die Menge des Additivs im Bereich von 0,2 bis 2 Gew.-% liegt, bezogen auf die Menge des Katalysators.

14. Verfahren nach einem oder mehreren der Ansprüche 2 bis 13, wobei die Menge des Lösungsmittels so gewählt wird, dass das Lösungsmittel 20 bis 98 Gew.-% der gesamten zu hydrierenden Lösung bildet, vorzugsweise wobei die Menge des Lösungsmittels so gewählt wird, dass das Lösungsmittel 50 bis 95 Gew.-% der gesamten zu hydrierenden Lösung bildet, weiter bevorzugt wobei die Menge des Lösungsmittels so gewählt wird, dass das Lösungsmittel 70 bis 90 Gew.-% der gesamten zu hydrierenden Lösung bildet.

15. Verfahren nach einem oder mehreren der vorhergehenden Ansprüche, wobei reines H₂-Gas oder eine Gasmischung, die H₂ umfasst, verwendet wird.

16. Verfahren nach einem oder mehreren der vorhergehenden Ansprüche, wobei der Katalysator mehrere Male wieder verwendet wird.

17. Verwendung einer Verbindung der Formel (II), mit der Maßgabe, dass R² nicht für Wasserstoff steht, wenn R¹ = Methyl und n = 1, als Aromabestandteile.

18. Verwendung einer Verbindung der Formel (II), mit der Maßgabe, dass R² nicht für Wasserstoff steht, wenn R¹ = Methyl und n = 1, bei Geschmacks- oder Duftstoffanwendungen.

19. Geschmacks- oder Duftstoffformulierung, umfassend
(i) mindestens eine Verbindung der Formel (II), mit der Maßgabe, dass R² nicht für Wasserstoff steht, wenn R¹ = Methyl und n = 1.

20. Geschmacks- und Duftstoffformulierung nach Anspruch 19, umfassend 0,0001-10 Gew.-% mindestens einer Verbindung der Formel (II), bezogen auf das Gesamtgewicht der Geschmacks- und Duftstoffformulierung.

21. Geschmacks- und Duftstoffformulierung nach Anspruch 19 und/oder 20, wobei die Geschmacks- und Duftstoffformulierung fest, gelartig oder flüssig ist.

22. Geschmacks- und Duftstoffformulierung nach einem oder mehreren der Ansprüche 19-21, wobei es sich bei der Geschmacks- und Duftstoffformulierung um ein Parfüm, Luftpflegeprodukt, Haushaltsprodukt, Waschmittelprodukt, Körperpflegeprodukt oder Kosmetikprodukt handelt.

23. Verfahren zur Verbesserung, Verstärkung oder Modifizierung einer Geschmacks- oder Duftstoffformulierung durch Versetzen mit einer olfaktorisch unbedenklichen Menge mindestens einer Verbindung der Formel (II), mit der Maßgabe, dass R² nicht für Wasserstoff steht, wenn R¹ = Methyl und n = 1.

24. Verbindung der Formel (II) 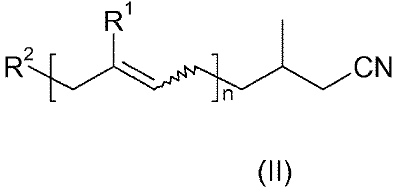 wobei n entweder für 1 oder für 2 steht, vorzugsweise wobei n = 1; und
wobei R¹ für lineares C₁₋₄-Alkyl oder verzweigtes C₃₋₄-Alkyl steht,
vorzugsweise wobei R¹ für Methyl oder Ethyl steht, und
wobei R² für Wasserstoff oder lineares C₁₋₄-Alkyl oder verzweigtes C₃₋₄-Alkyl steht,
vorzugsweise wobei R² für Wasserstoff oder Methyl steht,
mit der Maßgabe, dass R² nicht für Wasserstoff steht, wenn R¹ = Methyl und n = 1 oder 2.

## Revendications

1. Procédé de préparation d'un composé de formule II 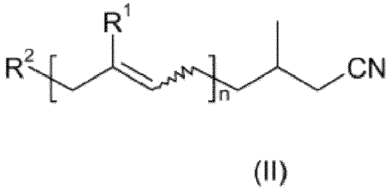 par réduction d'un composé de formule I 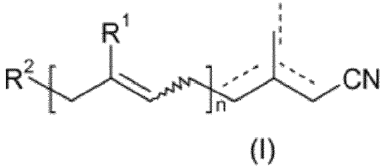 avec de l'hydrogène en présence d'un catalyseur comprenant du nickel, un additif étant présent et n étant 1 ou 2, préférablement n étant 1 ;
R¹ représentant C₁₋₄-alkyle linéaire ou C₃₋₄-alkyle ramifié,
préférablement R¹ représentant méthyle ou éthyle et
R² représentant hydrogène ou C₁₋₄-alkyle linéaire ou C₃₋₄-alkyle ramifié,
préférablement R² représentant hydrogène ou méthyle et
plus préférablement soit R¹ représentant méthyle soit R² représentant hydrogène ;
l'additif étant choisi dans le groupe d'additifs contenant un atome d'azote ou un atome de soufre,
l'additif contenant un atome de soufre étant choisi dans le groupe des disulfures aromatiques, des disulfures aliphatiques, des sulfures aromatiques, des sulfures hétéroaromatiques, des sulfures aliphatiques, des thiols aromatiques, des thiols hétéroaromatiques, des thiols aliphatiques, chacun contenant respectivement un nombre total d'atomes de carbone de 2 jusqu'à 15 et un nombre total d'atomes de soufre de 1 ou 2, et
l'additif contenant un atome d'azote étant choisi dans le groupe des amines primaires aliphatiques contenant un nombre total d'atomes de carbone de 3 jusqu'à 10 et 1-3 atome(s) d'azote et des amidines aliphatiques bicycliques en C₆₋₁₀ ; et la réduction avec de l'hydrogène étant mise en oeuvre à une pression absolue d'hydrogène dans la plage de 1 jusqu'à 20 bar(s).

2. Procédé selon la revendication 1, la réduction étant mise en oeuvre dans un solvant organique.

3. Procédé selon la revendication 2, le solvant organique étant choisi dans le groupe constitué par les hydrocarbures aliphatiques, les hydrocarbures aromatiques, les esters, les éthers et les alcools et les quelconques mélanges correspondants, préférablement le solvant organique étant choisi dans le groupe constitué par les hydrocarbures aliphatiques, les alcools et les quelconques mélanges correspondants.

4. Procédé selon la revendication 2 et/ou la revendication 3, le solvant organique étant le n-heptane ou le 2-propanol ou un quelconque mélange correspondant, préférablement le solvant organique étant le 2-propanol.

5. Procédé selon l'une quelconque des revendications précédentes, l'additif étant choisi dans le groupe d'additifs contenant un atome de soufre.

6. Procédé selon la revendication 1, l'additif contenant un atome d'azote étant choisi dans le groupe constitué par le 1,8-diazabicyclo[5.4.0]undéc-7-ène, l'éthylènediamine et le 1,4-diaminobutane.

7. Procédé selon la revendication 1, l'additif contenant un atome de soufre étant choisi dans le groupe constitué par le disulfure de diéthyle, le disulfure de diphényle, le sulfure de diéthyle, le sulfure de diphényle, le disulfure de diéthylène, le thiophène et le 2,2'-(éthylènedithio)-diéthanol, préférablement l'additif contenant un atome de soufre étant choisi dans le groupe constitué par le disulfure de diéthyle, le sulfure de diéthyle, le sulfure de diphényle, le thiophène et le 2,2'-(éthylènedithio)-diéthanol, plus préférablement l'additif contenant un atome de soufre étant le thiophène ou le 2,2'-(éthylènedithio)-diéthanol.

8. Procédé selon l'une quelconque des revendications précédentes, la réduction avec de l'hydrogène étant mise en oeuvre à une température dans la plage de 10 jusqu'à 100°C, préférablement à une température dans la plage de 20 jusqu'à 80°C, plus préférablement à une température dans la plage de 40 à 60°C, encore plus préférablement à une température dans la plage de 45 jusqu'à 55°C.

9. Procédé selon l'une quelconque des revendications précédentes, la réduction avec de l'hydrogène étant mise en oeuvre à une pression absolue d'hydrogène dans la plage de 2 à 11 bars, préférablement à une pression d'hydrogène dans la plage de 4 à 9 bars, plus préférablement à une pression absolue d'hydrogène de 6 bars.

10. Procédé selon l'une quelconque des revendications précédentes, le catalyseur étant du type alliage de nickel, préférablement le catalyseur comprenant une quantité de nickel dans la plage de 75 jusqu'à 95% en poids et une quantité d'aluminium dans la plage de 0-15% en poids, le reste étant constitué par d'autres métaux de sorte que le poids total s'élève à 100%.

11. Procédé selon la revendication 10, le catalyseur étant activé par du fer ou du chrome ou par les deux, préférablement en une quantité de 0 jusqu'à 10% en poids, sur la base du poids total du catalyseur.

12. Procédé selon l'une quelconque des revendications précédentes, la quantité de catalyseur se situant dans la plage de 1 jusqu'à 100% en poids, préférablement la quantité de catalyseur se situant dans la plage de 10 jusqu'à 80% en poids, plus préférablement la quantité de catalyseur se situant dans la plage de 30 jusqu'à 60% en poids, sur la base de la quantité du composé de formule (I) .

13. Procédé selon l'une quelconque des revendications précédentes, la quantité d'additif se situant dans la plage de 0,01 jusqu'à 100% en poids, préférablement la quantité d'additif se situant dans la plage de 0,1 jusqu'à 10% en poids, plus préférablement la quantité d'additif se situant dans la plage de 0,2 jusqu'à 2% en poids, sur la base de la quantité de catalyseur.

14. Procédé selon l'une quelconque des revendications 2 à 13, la quantité de solvant étant choisie de telle sorte que le solvant représente 20 jusqu'à 98% en poids de la solution totale à hydrogéner, préférablement la quantité de solvant étant choisie de telle sorte que le solvant représente 50 jusqu'à 95% en poids de la solution totale à hydrogéner, plus préférablement la quantité de solvant étant choisie de telle sorte que le solvant représente 70 jusqu'à 90% en poids de la solution totale à hydrogéner.

15. Procédé selon l'une quelconque des revendications précédentes, du gaz H₂ pur ou un mélange de gaz comprenant H₂ étant utilisé.

16. Procédé selon l'une quelconque des revendications précédentes, le catalyseur étant réutilisé plusieurs fois.

17. Utilisation d'un composé de formule (II), étant entendu que R² ne représente pas hydrogène si R¹ = méthyle et n = 1, en tant qu'ingrédient d'arôme.

18. Utilisation d'un composé de formule (II), étant entendu que R² ne représente pas hydrogène si R¹ = méthyle et n = 1, dans des applications de parfum et/ou de fragrance.

19. Formulation de parfum ou de fragrance comprenant
(i) au moins un composé de formule (II), étant entendu que R² ne représente pas hydrogène si R¹ = méthyle et n = 1.

20. Formulation de parfum et de fragrance selon la revendication 19 comprenant 0,0001-10% en poids d'au moins un composé de formule (II), sur la base du poids total de la formulation de parfum et de fragrance.

21. Formulation de parfum et de fragrance selon la revendication 19 et/ou la revendication 20, la formulation de parfum et de fragrance étant solide, similaire à un gel ou liquide.

22. Formulation de parfum et de fragrance selon l'une quelconque des revendications 19-21, la formulation de parfum et de fragrance étant un parfum, un produit d'assainissement de l'air, un produit ménager, un produit de buanderie, un produit de soin du corps ou un produit cosmétique.

23. Procédé d'amélioration, d'augmentation ou de modification d'une formulation de parfum ou de fragrance par ajout à celle-ci d'une quantité acceptable sur le plan olfactif d'au moins un composé de formule (II), étant entendu que R² ne représente pas hydrogène si R¹ = méthyle et n = 1.

24. Composé de formule (II) 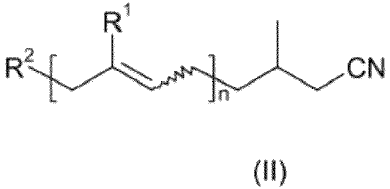 n étant 1 ou 2, préférablement n = 1 ; et
R¹ représentant C₁₋₄-alkyle linéaire ou C₃₋₄-alkyle ramifié,
préférablement R¹ représentant méthyle ou éthyle et
R² représentant hydrogène ou C₁₋₄-alkyle linéaire ou C₃₋₄-alkyle ramifié,
préférablement R² représentant hydrogène ou méthyle,
étant entendu que R² ne représente pas hydrogène si R¹ = méthyle et n = 1 ou 2.
